# EUROPEAN PATENT APPLICATION

(11) **EP 4 729 053 A1**
(43) Date of publication of application: **22.04.2026**
(21) Application number: 24822808.2
(22) Date of filing: 14.06.2024
(51) Int. Cl.: A61K 9/20, C07D 231/56

(54) **SOLID DISPERSION OF P2X4 INHIBITOR, AND PREPARATION METHOD THEREFOR AND USE THEREOF**

(30) Priority: 16.06.2023 CN 202310724887; 09.05.2024 CN 202410567332
(71) Applicant: Wuhan Createrna Science and Technology Co.,Ltd., Wuhan, Hubei 430075 (CN)
(72) Inventor: HONG, Huayun, Wuhan, Hubei 430075 (CN); WANG, Yali, Wuhan, Hubei 430075 (CN); LOU, Jun, Wuhan, Hubei 430075 (CN); ZHANG, Yihan, Wuhan, Hubei 430075 (CN)
(74) Representative: Michalski Hüttermann & Partner Patentanwälte mbB
(86) International application number: PCT/CN2024/099304
(87) International publication number: WO 2024/255861

(57) **Abstract**

A solid dispersion of a P2X4 inhibitor, comprising an active ingredient and a carrier. The obtained solid dispersion can improve the solubility and dissolution effect of the active ingredient, has a good stability, and has no obvious increase in impurities under 25°C/60% RH (open) and 40°C/75% RH (open) conditions. The method for preparing the solid dispersion is simple. The solid dispersion can be prepared using a spray drying method, a solvent method, or a hot-melt extrusion method, and the solvent residue content in the obtained product is qualified. Furthermore, the obtained solid dispersion effectively improves the pharmacokinetic effect of the active ingredient. A pharmaceutical composition containing the solid dispersion. The pharmaceutical composition has a good disintegration effect, and the disintegration time is within pharmacopoeial limits. Therefore, the pharmaceutical composition provides convenience for the development of subsequent dosage forms of the active ingredient.

## Description

The present application claims priority to the following prior applications: a prior application with patent application No. 2023107248878, entitled "SOLID DISPERSION OF P2X4 INHIBITOR, AND PREPARATION METHOD THEREFOR AND USE THEREOF" and filed with the China National Intellectual Property Administration on June 16, 2023; and a prior application with patent application No. 2024105673321, entitled "SOLID DISPERSION OF P2X4 INHIBITOR, AND PREPARATION METHOD THEREFOR AND USE THEREOF" and filed with the China National Intellectual Property Administration on May 9, 2024. The contents of these two prior applications are incorporated herein by reference in their entirety.

### TECHNICAL FIELD

The present disclosure belongs to the field of pharmaceutical technology, and specifically relates to a solid dispersion of a P2X4 inhibitor, and a preparation method therefor and use thereof.

### BACKGROUND

Purinergic receptors are widely present on the membranes of almost all mammalian cells and are involved in the regulation of multiple biological signaling pathways. Purinergic receptors can be classified into adenosine and ATP receptors. ATP receptors are broadly divided into the P2X family of ion channel receptors and the P2Y family of G protein-coupled receptors. P2X receptors are widely distributed in various tissues throughout the body, including the brain, lungs, neurons, glial cells, heart, blood vessels, *etc.* To date, seven subtypes of P2X receptors (P2X1 to 7) have been disclosed. Specifically, P2X4 is the only subtype in the P2X family for which the crystal structure has been resolved, with a resolution of up to 2.8 Å. Furthermore, studies have found that P2X4 is the P2X subtype with the highest permeability to Ca²⁺.

Currently, P2X4 inhibitors are the research focus for many companies.

Patent document WO2016198374A1 discloses several P2X4 inhibitor compounds, which are antagonists or negative allosteric modulators of P2X4. Such compounds are used for treating or preventing diseases related to pain, or for treating or preventing pain syndromes (acute and chronic), inflammation-induced pain, neuropathic pain, pelvic pain, cancer-related pain, endometriosis-related pain, as well as endometriosis itself, cancer itself, and proliferative diseases similar to endometriosis. They can be used as a single agent or in combination with other active components.

Patent document WO2017191000A1 discloses several P2X4 inhibitor compounds, which are used as the sole agent or in combination with other active ingredients for the manufacture of a pharmaceutical composition. The pharmaceutical composition is used for treating or preventing diseases (particularly diseases in mammals, such as, but not limited to, diseases related to pain), or for treating or preventing pain or neuronal damage and inflammation in the brain or bone marrow, arthritis or spondylitis syndromes (acute and chronic), inflammation-induced pain, neuropathic pain, pelvic pain, cancer-related pain, endometriosis-related pain, as well as endometriosis itself, cancer itself, multiple sclerosis itself, and bone marrow or ischemic brain injury itself.

Patent document WO2019081573A1 discloses several P2X4 inhibitor compounds for use in the manufacture of a medicament for treating hemorrhagic stroke and traumatic brain injury.

Patent documents WO2022002859A1 and WO2022002860A1 disclose several phenethylamide compounds with P2X4 receptor antagonistic activity, as well as the use of several phenethylamide compounds with P2X4 receptor antagonistic activity in the manufacture of a medicament for treating certain ocular diseases.

Patent document WO2021104486A1 discloses a benzene ring-containing compound and use thereof. Specifically, disclosed are several benzene ring-containing compounds as P2X4 inhibitors. Further disclosed are these compounds for use in the treatment of P2X4-mediated diseases. Also disclosed are compounds 1 to 48, which exhibit high inhibitory activity against P2X4, good selectivity, low toxicity, and good metabolic stability. However, such compounds have poor dissolution effects, making it necessary to develop formulations for these compounds to address the aforementioned issues.

### SUMMARY

The objective of the present disclosure is to address the deficiencies in the prior art by providing a solid dispersion of a P2X4 inhibitor, comprising: an active ingredient and a carrier; the active ingredient is at least one selected from the group consisting of the following compounds 1 to 48 or a pharmaceutically acceptable salt thereof: 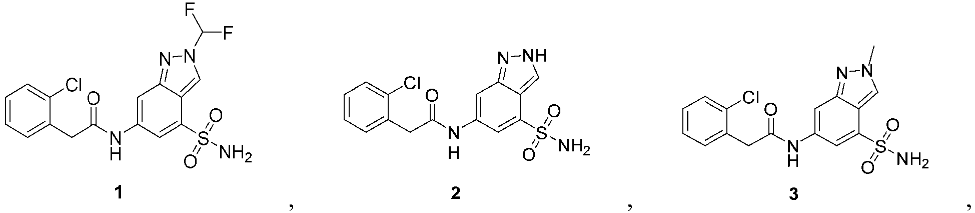 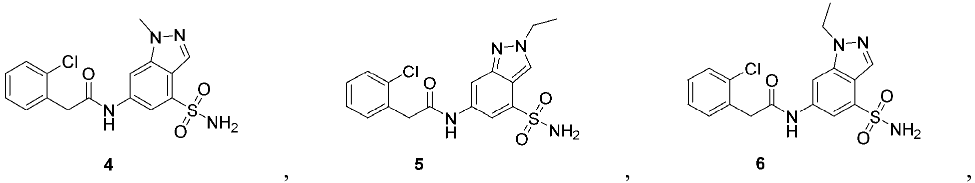 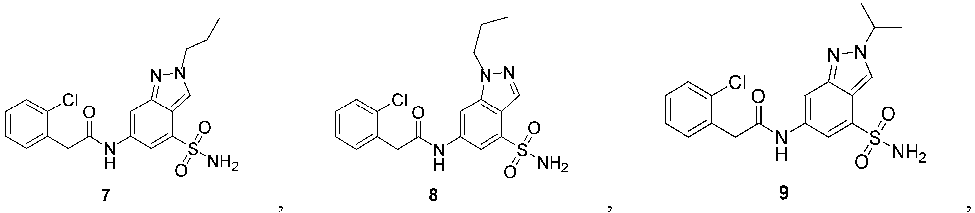 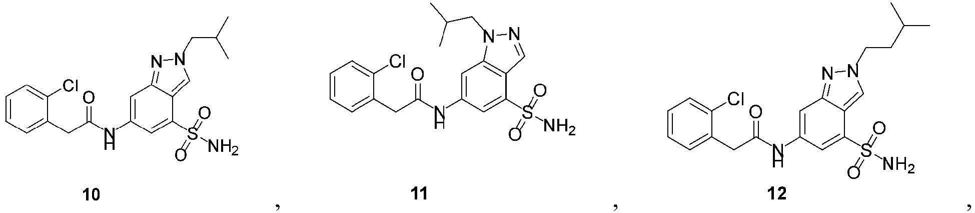 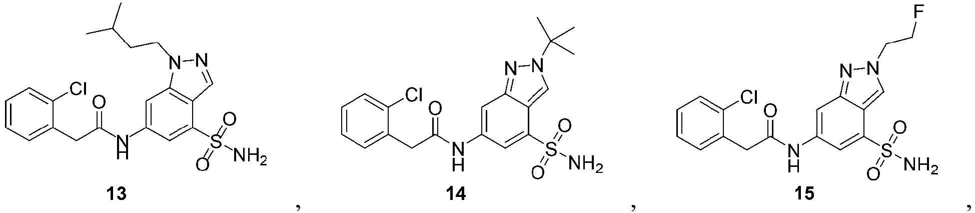 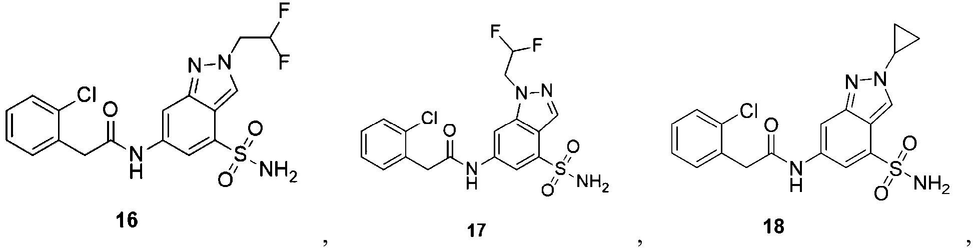 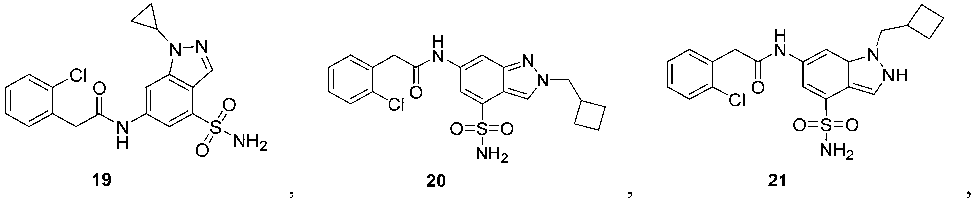 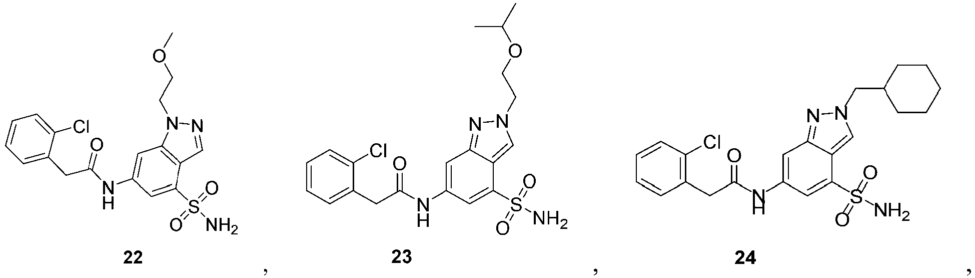 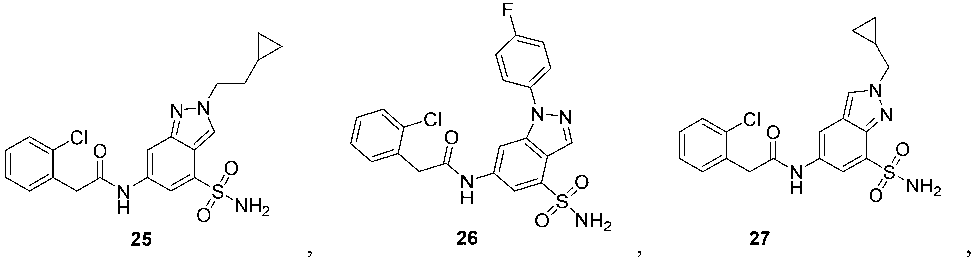 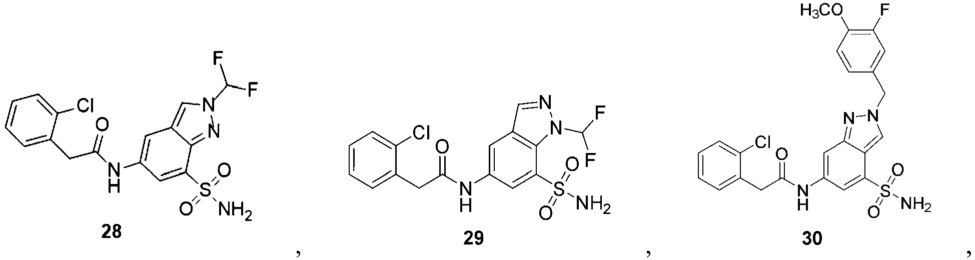 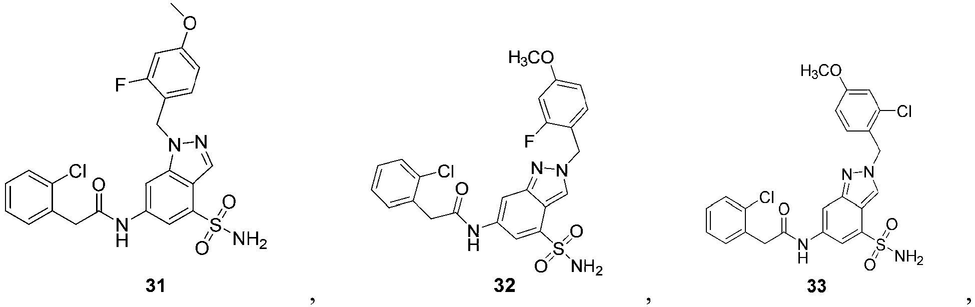 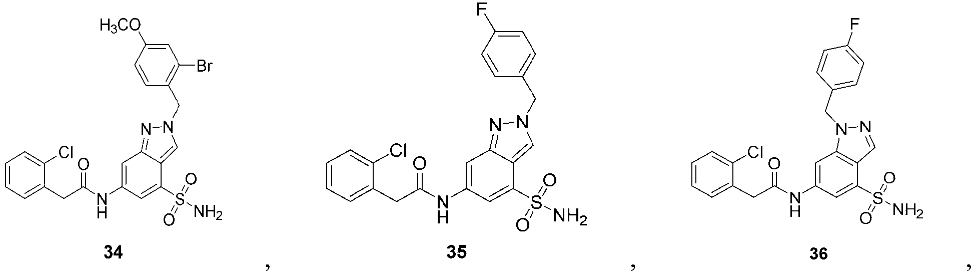 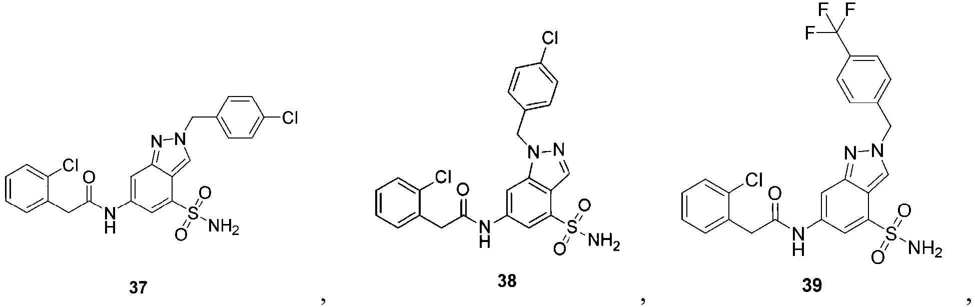 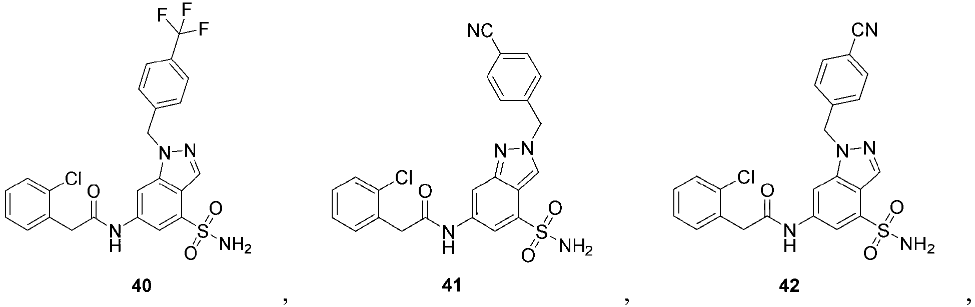 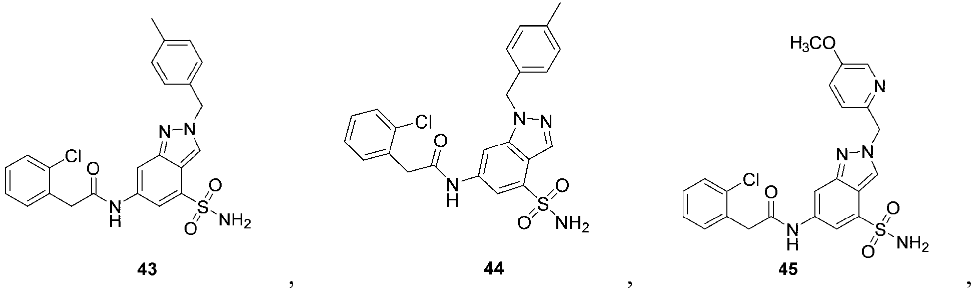 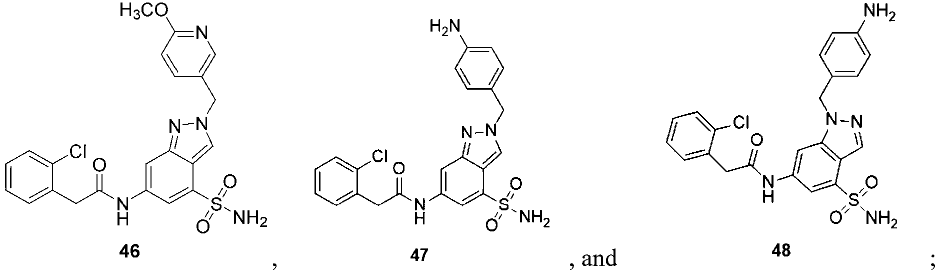 the carrier is one or more selected from the group consisting of copovidone, povidone, hypromellose acetate succinate, hypromellose phthalate (HPMPCP), polyvinyl caprolactam-polyvinyl acetate-polyethylene glycol graft copolymer (Soluplus), hydroxypropyl cellulose, hypromellose, polyacrylic resin, cellulose acetate phthalate (CAP), methacrylic acid-methyl methacrylate copolymer, and polyethylene oxide (PEO), preferably copovidone, hypromellose acetate succinate, or povidone, and most preferably copovidone.

According to an embodiment of the present disclosure, in the carrier, the copovidone is PVP-VA64, wherein copovidone is also known as polyvinylpyrrolidone/vinyl acetate copolymer, which is a series of linear copolymers of N-vinylpyrrolidone (NVP) and vinyl acetate (VA), and commercially available copovidones include: PVP-VA64, Kollidon VA64, Plasdone S-630, *etc.;* the povidone is PVP-K30, wherein povidone is a series of homopolymers of water-soluble N-vinylpyrrolidone (NVP), and commonly used povidones include PVP-K30, PVP-K90, *etc.;* the hypromellose is HPMC E3; the polyacrylic resin is Eudragit L100.

According to an embodiment of the present disclosure, the solid dispersion further comprises a surfactant.

According to an embodiment of the present disclosure, the surfactant is one selected from the group consisting of sodium lauryl sulfate (abbreviated as SLS or SDS), sodium cetyl sulfate, sodium stearyl sulfate, Tween (*e.g.,* Tween 80, 60, 40, and 20), hydrogenated castor oil (*e.g.,* Cremophor EL), glyceryl monostearate, vitamin E polyethylene glycol succinate (VE-TPGS), polyoxyethylene castor oil derivative (*e.g.,* Cremophor RH40), Poloxamer (*e.g.,* Poloxamer F68, Poloxamer 124, Poloxamer 188, Poloxamer 237, Poloxamer 338, and Poloxamer 407), and polyethylene glycol-15 hydroxystearate (Solutol HS15), or a mixture thereof obtained by mixing two or more thereof in any proportion, preferably one selected from the group consisting of sodium lauryl sulfate, vitamin E polyethylene glycol succinate, and Cremophor RH40, or a mixture thereof obtained by mixing two or more thereof in any proportion.

In a preferred embodiment, the active ingredient is compound 1 or a pharmaceutically acceptable salt thereof.

According to an embodiment of the present disclosure, compounds 1 to 48 may refer to the compounds disclosed in the patent document WO2021104486A1, the contents of which are incorporated herein by reference in their entirety.

According to an embodiment of the present disclosure, the active ingredient and the carrier have a mass ratio of 1:(1 to 7), preferably 1:(2 to 5), more preferably 3:(6 to 7), and most preferably 3:7.

According to some embodiments of the present disclosure, the active ingredient and the carrier have a mass ratio of 1:(1 to 7), preferably 1:(2 to 5), more preferably 3:(6 to 9), and most preferably 1:2.8.

According to an embodiment of the present disclosure, the solid dispersion comprises: an active ingredient, a carrier, and a surfactant; the active ingredient, the carrier, and the surfactant have a mass ratio of 1:(1 to 7):(0.1 to 1), preferably 1:(2 to 5):(0.1 to 1), such as 3:6.5:0.5.

In some embodiments of the present disclosure, the solid dispersion comprises: compound 1 and copovidone;
or, compound 1 and povidone;
or, compound 1 and hypromellose acetate succinate;
or, compound 1, copovidone, and Cremophor RH40;
or, compound 1, copovidone, and SLS;
or, compound 1, copovidone, and VE-TPGS;
or, compound 1, povidone, and Cremophor RH40;
or, compound 1, povidone, and SLS;
or, compound 1, povidone, and VE-TPGS.

In some embodiments of the present disclosure, the solid dispersion comprises: compound 1, copovidone, and Cremophor RH40;
or, compound 1, copovidone, and SLS;
or, compound 1, copovidone, and VE-TPGS;
or, compound 1, povidone, and Cremophor RH40;
or, compound 1, povidone, and SLS;
or, compound 1, povidone, and VE-TPGS.

In some embodiments of the present disclosure, the solid dispersion comprises: compound 1, copovidone, and Cremophor RH40;
or, compound 1, copovidone, and SLS;
or, compound 1, copovidone, and VE-TPGS.

In some embodiments of the present disclosure, the solid dispersion comprises: compound 1, copovidone, and SLS;
or, compound 1, copovidone, and vitamin E polyethylene glycol succinate (VE-TPGS).

In some specific embodiments of the present disclosure, the solid dispersion comprises: compound 1, PVP-VA64, and SLS;
or, compound 1, PVP-VA64, and vitamin E polyethylene glycol succinate (VE-TPGS).

In some specific embodiments of the present disclosure, the solid dispersion comprises the following components in parts by weight: 1 part by weight of compound 1, 1 to 7 parts by weight of a carrier, and 0.1 to 1 part by weight of a surfactant, wherein the carrier is one or more selected from the group consisting of copovidone, povidone, and hypromellose acetate succinate, and the surfactant is one or more selected from the group consisting of sodium lauryl sulfate, vitamin E polyethylene glycol succinate (VE-TPGS), and polyoxyethylene castor oil derivative (*e*.*g*., Cremophor RH40).

In some specific embodiments of the present disclosure, the solid dispersion comprises the following components in parts by weight: 1 part by weight of compound 1, 1 to 7 parts by weight of PVP-VA64, and 0.1 to 1 part by weight of SLS; or, 1 part by weight of compound 1, 1 to 7 parts by weight of PVP-VA64, and 0.1 to 1 part by weight of vitamin E polyethylene glycol succinate (VE-TPGS).

The present disclosure further provides a preparation method for the solid dispersion as described above, wherein the solid dispersion is prepared using a spray drying method, a solvent method, or a hot-melt extrusion method, comprising the following steps:
spray drying method: adding a raw material to a solvent S1 to prepare a solution, followed by spray drying; in a formulation comprising a surfactant, adding the active ingredient, the carrier, and the surfactant to the solvent S1 to prepare a solution, followed by spray drying;
solvent method: adding a raw material to a solvent S2 and stirring uniformly until the raw material is completely dissolved to obtain a solution, then removing the solvent from the resulting solution, followed by drying and pulverizing to obtain the solid dispersion;
hot-melt extrusion method: mixing a raw material, and optionally adding an appropriate amount of lubricant for hot-melt extrusion;
the solvent S1 is a ketone solvent, or a mixed solvent composed of a ketone solvent and a solvent selected from the group consisting of a halogenated alkane solvent, water, and an alcohol solvent;
the solvent S2 is a ketone solvent, or a mixed solvent composed of a ketone solvent and a solvent selected from the group consisting of a halogenated alkane solvent and an alcohol solvent.

In the preparation method for the solid dispersion, the "raw material" comprises an active ingredient and a carrier; when the solid dispersion further comprises a surfactant, the raw material comprises the active ingredient, the carrier, and the surfactant.

According to an embodiment of the present disclosure, the raw material in the preparation method for the solid dispersion may comprise compound 1 as the active ingredient and a carrier, or may comprise compound 1 as the active ingredient, a carrier, and optionally a surfactant, depending on the specific requirements for preparing the solid dispersion;
The term "optionally" includes no selection, or selection of one, two, or more of the foregoing components.

According to an embodiment of the present disclosure, the ketone solvent may be one or more selected from the group consisting of acetone, butanone, and methyl isobutyl ketone, preferably acetone.

According to an embodiment of the present disclosure, the halogenated alkane solvent is dichloromethane.

According to an embodiment of the present disclosure, the alcohol solvent is methanol, ethanol, or propanol.

According to an embodiment of the present disclosure, the solvent S1 is a binary mixed solvent composed of a ketone solvent and a solvent selected from the group consisting of a halogenated alkane solvent, water, and an alcohol solvent, wherein the ketone solvent and the other solvent have a volume ratio of (1 to 10):1, such as (1 to 5):1.

According to an embodiment of the present disclosure, the solvent S1 is a binary mixed solvent composed of acetone and a solvent selected from the group consisting of dichloromethane, ethanol, and water, wherein the acetone and the other solvent have a volume ratio of (1 to 5):1.

According to an embodiment of the present disclosure, the solvent S1 is a binary mixed solvent of acetone and dichloromethane, and the volume ratio of acetone to dichloromethane is 6:4; or, the solvent S1 is a binary mixed solvent of acetone and water, and the volume ratio of acetone to water is 7:3; or, the solvent S1 is a binary mixed solvent of acetone and ethanol, and the volume ratio of acetone to ethanol is 1:1.

According to an embodiment of the present disclosure, the solvent S1 is a mixed solvent composed of acetone, ethanol, and water.

According to a specific embodiment of the present disclosure, the solvent S1 is a mixed solvent composed of acetone, ethanol, and water, wherein the volume ratio of acetone, ethanol, and water is 1:(0.8 to 1):(0.2 to 0.7), preferably 8:7:5, 2:2:1, 5:4:1, or 4:3:3, and most preferably 8:7:5.

According to an embodiment of the present disclosure, when the solvent S2 is a binary mixed solvent, the volume ratio of the ketone solvent to the other solvent is (1 to 10):1, such as (5 to 9):1.

According to an embodiment of the present disclosure, when the spray drying method is used, the concentration of the active ingredient in the resulting solution is 5 to 50 mg/mL, such as 15 to 25 mg/mL.

According to an embodiment of the present disclosure, when the solvent method is used, the mass fraction of the active ingredient in the resulting solution is 5% to 50%, such as 10% to 40%.

According to an embodiment of the present disclosure, in the method for preparing the solid dispersion by hot-melt extrusion, the lubricant is one selected from the group consisting of talc, magnesium stearate, calcium stearate, colloidal silicon dioxide, hydrated silicon dioxide, hydrophobic silicon dioxide, sodium stearyl fumarate, polyethylene glycol, sodium stearyl fumarate, glyceryl monostearate, and hydrogenated vegetable oil, or a mixture thereof obtained by mixing two or more thereof in any proportion.

The present disclosure further provides a pharmaceutical composition comprising the solid dispersion as described above and a pharmaceutically acceptable excipient.

According to an embodiment of the present disclosure, the pharmaceutical composition is formulated into a tablet, a powder, a granule, a capsule, a drop pill, or a pellet, preferably a tablet by a conventional preparation process.

The route of administration of the pharmaceutical composition of the present disclosure includes oral, rectal, vaginal, and sublingual administration.

According to an embodiment of the present disclosure, the excipient comprises one or more selected from the group consisting of a filler, a lubricant, and a disintegrant.

According to an embodiment of the present disclosure, the filler is one selected from the group consisting of lactose, dextrin, mannitol, microcrystalline cellulose, starch, pregelatinized starch, cellulose lactose, dicalcium phosphate, and a mannitol-starch complex, or a mixture thereof obtained by mixing two or more thereof in any proportion, preferably one selected from the group consisting of microcrystalline cellulose, mannitol, and starch, or a mixture thereof obtained by mixing two or more thereof in any proportion.

According to an embodiment of the present disclosure, the lubricant is one selected from the group consisting of talc, magnesium stearate, calcium stearate, colloidal silicon dioxide, hydrated silicon dioxide, hydrophobic silicon dioxide, sodium stearyl fumarate, polyethylene glycol, sodium stearyl fumarate, glyceryl monostearate, and hydrogenated vegetable oil, or a mixture thereof obtained by mixing two or more thereof in any proportion; preferably one selected from the group consisting of magnesium stearate, colloidal silicon dioxide, and hydrophobic silicon dioxide, or a mixture thereof obtained by mixing two or more thereof in any proportion.

According to an embodiment of the present disclosure, the disintegrant is one selected from the group consisting of dry starch, croscarmellose sodium, sodium carboxymethylcellulose, calcium carboxymethylcellulose, sodium carboxymethyl starch, methylcellulose, low-substituted hydroxypropyl cellulose, crospovidone, chitosan, and microcrystalline cellulose, or a mixture thereof obtained by mixing two or more thereof in any proportion; preferably one selected from the group consisting of croscarmellose sodium, sodium carboxymethylcellulose, and calcium carboxymethylcellulose, or a mixture thereof obtained by mixing two or more thereof in any proportion.

According to an embodiment of the present disclosure, the pharmaceutical composition comprises, by weight percentage, 30% to 50% of the solid dispersion, 20% to 50% of the filler, 1% to 15% of the disintegrant, and 1% to 15% of the lubricant;
preferably, the pharmaceutical composition comprises, by weight percentage, 35% to 45% of the solid dispersion, 35% to 45% of the filler, 1% to 10% of the disintegrant, and 1% to 10% of the lubricant;
more preferably, the pharmaceutical composition comprises, by weight percentage, 40% to 45% of the solid dispersion, 40% to 45% of the filler, 5% to 10% of the disintegrant, and 1% to 5% of the lubricant.

According to an embodiment of the present disclosure, the pharmaceutical composition further comprises a disintegration promoter.

According to an embodiment of the present disclosure, the disintegration promoter is one selected from the group consisting of sodium chloride, sodium carbonate, calcium carbonate, potassium carbonate, calcium magnesium carbonate, zinc carbonate, magnesium carbonate, ammonium carbonate, sodium glycine carbonate, sodium sesquicarbonate, sodium bicarbonate, calcium bicarbonate, potassium bicarbonate, and ammonium bicarbonate, or a mixture thereof obtained by mixing two or more thereof in any proportion; preferably one selected from the group consisting of sodium chloride, sodium carbonate, and sodium bicarbonate, or a mixture thereof obtained by mixing two or more thereof in any proportion.

According to an embodiment of the present disclosure, when the disintegration promoter is further included, the pharmaceutical composition comprises, by weight percentage, 30% to 50% of the solid dispersion, 20% to 50% of the filler, 1% to 15% of the disintegrant, 1% to 15% of the lubricant, and 0.1% to 10% of the disintegration promoter;
preferably, the pharmaceutical composition comprises, by weight percentage, 35% to 45% of the solid dispersion, 35% to 45% of the filler, 1% to 10% of the disintegrant, 1% to 10% of the lubricant, and 1% to 10% of the disintegration promoter;
more preferably, the pharmaceutical composition comprises, by weight percentage, 40% to 45% of the solid dispersion, 40% to 45% of the filler, 5% to 10% of the disintegrant, 1% to 5% of the lubricant, and 5% to 10% of the disintegration promoter.

According to an embodiment of the present disclosure, the pharmaceutical composition is a solid dosage form, such as a tablet.

The present disclosure further provides a preparation method for the pharmaceutical composition as described above, comprising the step of mixing the solid dispersion with a pharmaceutically acceptable excipient.

According to an embodiment of the present disclosure, the excipient may be sieved prior to mixing.

According to an embodiment of the present disclosure, the pharmaceutical composition is prepared by a dry granulation process, wherein the preparation method comprises: weighing a formulation amount of materials, wherein the solid dispersion is not sieved, while the other excipients are sieved for later use; pre-mixing the intra-granular filler, disintegrant, glidant, and solid dispersion, followed by milling and sieving; adding the lubricant for dry granulation; then adding the extra-granular filler, disintegrant, swelling agent, and lubricant, followed by final blending. Preferably, after final blending, the mixture is packaged according to the required amount of the active ingredient, and then compressed into tablets of pharmaceutical specifications.

The active ingredient used in the present disclosure exhibits inhibitory, antagonistic, and negative allosteric modulatory effects on the P2X4 receptor. Therefore, administration of a certain amount of the solid dispersion or pharmaceutical composition of the present disclosure to a mammal (including a human) exhibits inhibitory, antagonistic, and negative allosteric modulatory effects on the P2X4 receptor, thereby effectively treating related disorders.

The present disclosure further provides the use of the solid dispersion or pharmaceutical composition as described above in the manufacture of a P2X4 receptor antagonist.

According to an embodiment of the present disclosure, the P2X4 receptor antagonist is used to treat the following diseases: a respiratory system disease, a urinary system disease, a gynecological disease, an ophthalmic disease, a gastrointestinal disease, a proliferative disease, a cardiovascular and cerebrovascular disease, a neurodegenerative or degenerative disease, inflammation, a pain-related disease, pruritus (e.g., chronic pruritus), stroke, ischemic brain injury, traumatic brain injury, a musculoskeletal and connective tissue disorder, or a systemic disorder;
the respiratory system disease is, for example, a respiratory disorder, including idiopathic pulmonary fibrosis, respiratory failure, chronic obstructive pulmonary disease, asthma, bronchospasm, cough (e.g., acute cough, chronic cough), refractory chronic cough, or idiopathic chronic cough;
the urinary system disease is, for example, urinary incontinence, overactive bladder, dysuria, prostatic hyperplasia, cystitis (e.g., interstitial cystitis), bladder pain syndrome, or prostatitis;
the gynecological disease is, for example, endometriosis, primary and secondary dysmenorrhea, dyspareunia, or adenomyosis;
the ophthalmic disease is, for example, dry eye syndrome, ocular neuropathic pain, ocular trauma, postoperative ocular pain, and the like;
the gastrointestinal disease is, for example, gastrointestinal dysfunction, irritable bowel syndrome (IBS), inflammatory bowel disease (IBD), Crohn's disease, gastroesophageal reflux, and the like;
the proliferative disease is, for example, a tumor, such as cancer;
the cardiovascular disease is, for example, myocardial infarction, thrombosis, atherosclerosis, heart failure, or hypertension;
the neurodegenerative or degenerative disease is, for example, Parkinson's disease, Alzheimer's disease, Huntington's disease, amyotrophic lateral sclerosis, stroke, ischemic brain injury, or traumatic brain injury;
the inflammation is, for example, arthritis, rheumatoid arthritis, ankylosing spondylitis and related neuropathic pain, or gout;
the pain-related disease is, for example, hyperalgesia, allodynia, acute and chronic inflammatory and neuropathic pain, inflammatory pain, postoperative pain, visceral pain, toothache, periodontitis, premenstrual pain, endometriosis-related pain, fibrosis-related pain, central pain, burn pain, or migraine;
the pain-related disease is, for example, pain due to neuritis, pain due to poisoning, pain due to ischemic injury, pain due to interstitial cystitis, pain due to cancer, or pain due to traumatic nerve injury.

The present disclosure further provides the use of the solid dispersion or pharmaceutical composition as described above in the manufacture of a medicament for treating a P2X4-mediated disease.

The P2X4-mediated disease comprises a respiratory system disease, a urinary system disease, a gynecological disease, an ophthalmic disease, a gastrointestinal disease, a proliferative disease, a cardiovascular and cerebrovascular disease, a neurodegenerative or degenerative disease, inflammation, a pain-related disease, pruritus (e.g., chronic pruritus), stroke, ischemic brain injury, traumatic brain injury, a musculoskeletal and connective tissue disorder, or a systemic disorder;
the respiratory system disease is, for example, a respiratory disorder, including idiopathic pulmonary fibrosis, respiratory failure, chronic obstructive pulmonary disease, asthma, bronchospasm, cough (e.g., acute cough, chronic cough), refractory chronic cough, or idiopathic chronic cough;
the urinary system disease is, for example, urinary incontinence, overactive bladder, dysuria, prostatic hyperplasia, cystitis (e.g., interstitial cystitis), bladder pain syndrome, or prostatitis;
the gynecological disease is, for example, endometriosis, primary and secondary dysmenorrhea, dyspareunia, or adenomyosis;
the ophthalmic disease is, for example, dry eye syndrome, ocular neuropathic pain, ocular trauma, postoperative ocular pain, and the like;
the gastrointestinal disease is, for example, gastrointestinal dysfunction, irritable bowel syndrome (IBS), inflammatory bowel disease (IBD), Crohn's disease, gastroesophageal reflux, and the like;
the proliferative disease is, for example, a tumor, such as cancer;
the cardiovascular disease is, for example, myocardial infarction, thrombosis, atherosclerosis, heart failure, or hypertension;
the neurodegenerative or degenerative disease is, for example, Parkinson's disease, Alzheimer's disease, Huntington's disease, amyotrophic lateral sclerosis, stroke, ischemic brain injury, or traumatic brain injury;
the inflammation is, for example, arthritis, rheumatoid arthritis, ankylosing spondylitis and related neuropathic pain, or gout;
the pain-related disease is, for example, hyperalgesia, allodynia, acute and chronic inflammatory and neuropathic pain, inflammatory pain, postoperative pain, visceral pain, toothache, periodontitis, premenstrual pain, endometriosis-related pain, fibrosis-related pain, central pain, burn pain, or migraine;
the pain-related disease is, for example, pain due to neuritis, pain due to poisoning, pain due to ischemic injury, pain due to interstitial cystitis, pain due to cancer, or pain due to traumatic nerve injury.

The present disclosure further provides a method for treating a respiratory system disease, a urinary system disease, a gynecological disease, an ophthalmic disease, a gastrointestinal disease, a proliferative disease, a cardiovascular and cerebrovascular disease, a neurodegenerative or degenerative disease, inflammation, a pain-related disease, pruritus (e.g., chronic pruritus), stroke, ischemic brain injury, traumatic brain injury, a musculoskeletal and connective tissue disorder, or a systemic disorder, comprising administering the solid dispersion or pharmaceutical composition as described above to an individual in need thereof, the specific scope of these diseases being as described above.

### Advantageous effects

The present disclosure provides a solid dispersion comprising an active ingredient (compounds 1 to 48). The obtained solid dispersion can improve the solubility and dissolution effect of the active ingredient, has a good stability, and has no obvious increase in impurities under 25°C/60% RH (open) and 40°C/75% RH (open) conditions. Moreover, the preparation method is simple and can be implemented by a spray drying method, a solvent method, or a hot-melt extrusion method, with the resulting product meeting the requirements for residual solvents.

Furthermore, the obtained solid dispersion effectively improves the pharmacokinetic effect of the active ingredient.

The present disclosure further provides a pharmaceutical composition containing the solid dispersion. The pharmaceutical composition has a good disintegration effect, with the disintegration time within the pharmacopoeial limits. Therefore, the pharmaceutical composition of the present disclosure facilitates the further development of dosage forms of the active ingredient.

In the present disclosure, "several" means two or more.

### DETAILED DESCRIPTION OF THE PREFERRED EMBODIMENT

The present disclosure is further illustrated below by way of examples, but is not limited to the scope of the examples described herein. The experimental methods without specified conditions in the following examples are selected according to conventional methods and conditions, or according to product instructions.

The preparation methods and uses of the present disclosure will be further described in detail below with reference to specific examples. The following examples are merely illustrative and explanatory of the present disclosure and should not be construed as limiting the scope of protection of the present disclosure. All technologies implemented based on the foregoing contents of the present disclosure are encompassed within the scope intended to be protected by the present disclosure.

Unless otherwise specified, the raw materials and reagents used in the following examples are commercially available or can be prepared by known methods.

### Related reagents and their sources:

| Name | Manufacturer |
|---|---|
| FaSSIF/FaSSGF/FeSSIF powder | Biorelevant.com Ltd |
| Pancreatin | Sigma-Aldrich |
| Polyvinylpyrrolidone/vinyl acetate copolymer VA64 (PVP-VA64) | BASF SE, Germany |
| Hypromellose acetate succinate AS-MG (HPMCAS MG) | Shin-Etsu Silicone International Trading (Shanghai) Co., Ltd. |
| Polyvinylpyrrolidone K30 (PVP-K30) | BASF SE, Germany |
| Vitamin E polyethylene glycol succinate (VE-TPGS or TPGS) | Hisky (Beijing) Medical Technologies Co., Ltd. |
| Sodium lauryl sulfate (SLS) | BASF SE, Germany |
| Polyoxyl 40 hydrogenated castor oil (Cremophor RH40 or RH40) | BASF SE, Germany |
| N-methylpyrrolidone (NMP) | TCI (Shanghai) Chemical Industry Development |
| | Co., Ltd. |
| Microcrystalline cellulose | DuPont Nutrition USA, Inc |
| Mannitol | Merck KGaA, Darmstadt, Germany |
| Croscarmellose sodium | DuPont Nutrition USA, Inc |
| Crospovidone | ISP Chemical LLC Affiliate of Ashland Inc. |
| Magnesium stearate | Peter Greven Nederland C.V |
| Colloidal silicon dioxide | Evonik Operations GmbH |
| Hydrophobic silicon dioxide | Evonik Operations GmbH |

The following English abbreviations in the examples have the following meanings:
Cremophor EL: polyoxyethylene castor oil; Poloxamer 407: Poloxamer 407; Poloxamer 188: Poloxamer 188; Tween 80: Tween 80; HPC-SSL: hydroxypropyl cellulose; Eudragit L100: polyacrylic resin L100; SLS: sodium lauryl sulfate, sodium coco-sulfate, sodium dodecyl sulfate; Soluplus: polyvinyl caprolactam-polyvinyl acetate-polyethylene glycol graft copolymer; HPMC E3: hypromellose E3; TFA: trifluoroacetic acid; Avg. Dis: mean; RSD: relative standard deviation; FA: fatty acid.

### Experimental methods

### HPLC method (content and related substances)

| | | | |
|---|---|---|---|
| Instrument | Agilent 1260 series HPLC system with DAD detector | | |
| Chromatographic column | X Bridge Shield RP18 (4.6 * 150 mm, 3.5 µm) | | |
| Mobile phase A | 0.1% TFA aqueous solution (*v*/*v*) | | |
| Mobile phase B | 100% acetonitrile (*v*/*v*) | | |
| Column temperature | 30°C | | |
| Detection wavelength | 237 nm | | |
| Flow rate | 1.0 mL/min | | |
| Standard | 0.5 mg/mL | | |
| Injection volume | 2 µL | | |
| Diluent | Acetonitrile/water = 1/1 (*v*/*v*) | | |
| Elution procedure | Time (min) | Mobile phase A (%) | Mobile phase B (%) |
| | 0.00 | 90 | 10 |
| | 2.00 | 90 | 10 |
| | 20.00 | 50 | 50 |
| | 25.00 | 20 | 80 |
| | 30.00 | 20 | 80 |
| | 30.50 | 90 | 10 |
| | 35.00 | 90 | 10 |

### UPLC method (two-step dissolution)

| | | | |
|---|---|---|---|
| Instrument | Agilent 1290 Infinity II with DAD detector | | |
| Chromatographic column | Acquity UPLC BEH C18 | | |
| Mobile phase A | 0.1% TFA aqueous solution (*v*/*v*) | | |
| Mobile phase B | 100% acetonitrile (*v*/*v*) | | |
| Column temperature | 30°C | | |
| Detection wavelength | 237 nm | | |
| Flow rate | 0.3 mL/min | | |
| Standard | 0.5 mg/mL | | |
| Injection volume | 1 µL | | |
| Diluent | Acetonitrile/water = 1/1 (*v*/*v*) | | |
| Elution procedure | Time (min) | Mobile phase A (%) | Mobile phase B (%) |
| | 0 | 90 | 10 |
| | 1 | 90 | 10 |
| | 4 | 50 | 50 |
| | 6 | 20 | 80 |
| | 7 | 20 | 80 |
| | 7.01 | 90 | 10 |
| | 9 | 90 | 10 |

### Polarized light microscopy (PLM)

The details of the polarized light microscopy used in the testing are as follows: Instrument model: Nikon LV100POL polarized light microscope; Testing method: The sample is placed on a glass slide and dispersed in silicone oil before observation; Eyepiece: 50X; Objective: 5 to 50X.

### X-ray powder diffraction (XRPD)

The solid sample is characterized using a Bruker D8 advance X-ray diffractometer.

The sample is analyzed by XRPD under the following conditions:
X-ray tube: Cu:K-Alpha (λ = 1.54179 Å), tube voltage: 40 kV, tube current: 40 mA, scanning range: 3 to 40 deg., sample stage rotation: 15 rpm, scanning rate: 10 deg./min.

### Thermogravimetric analysis (TGA)

Instrument model: TA Q5000 thermogravimetric analyzer; the sample (2 to 10 mg) is placed in an aluminum pan and analyzed as follows: the sample is placed in a TGA aluminum pan and analyzed by heating from room temperature to 300°C (or until a 20% weight loss was observed) at a heating rate of 10°C/min under a 25 mL/min N₂ atmosphere.

### Differential scanning calorimetry (DSC)

Instrument model: TA Q2000 differential scanning calorimeter. The details of the DSC method used in the experiment are as follows: the sample (approximately 4 mg) is placed in a DSC aluminum pan with a perforated lid, and analyzed by heating from room temperature to 250°C at a heating rate of 2°C/min under a 50 mL/min N₂ atmosphere.

### Example 1: Preparation of compound 1

Compound 1 was prepared with reference to Example 1 of the published patent WO2021104486A1.

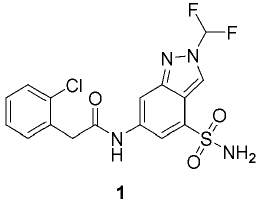

### Example 2: Solubilization test of compound 1 with surfactants

50 mg of compound 1 was weighed into 5 mL of FaSSIF medium containing 5 mg of different surfactants (target concentration of API (compound 1): 10 mg/mL). The mixture was shaken at 500 rpm and 37°C. 0.5 mL each of the suspension samples was taken at 0.5 hours, 1 hour, 2 hours, and 4 hours, respectively, centrifuged at 14,000 rpm for 5 minutes, and the supernatant was diluted and analyzed by HPLC. The test results are shown in Table 1.

**Table 1: Solubility results of compound 1 in FaSSIF containing 10% of surfactant by weight**

| Type of surfactant | Amount of surfactant (mg) | Amount of API (mg) | Vehicle FaSSIF (pH 6.5) (mL) | Concentration of API in vehicle (µg/mL) | | | |
|---|---|---|---|---|---|---|---|
| | | | | 0.5 h | 1 h | 2 h | 4 h |
| - | - | 50 | 5 | 33.9 | 22.9 | 15.2 | 14.9 |
| SLS | 5 | 50 | 5 | 614.0 | 622.0 | 639.0 | 652.0 |
| Cremophor EL | 5 | 50 | 5 | 1574.3 | 1604.3 | 1538.6 | 1508.2 |
| Cremophor RH40 | 5 | 50 | 5 | 1583.3 | 1604.5 | 1567.0 | 1539.4 |
| VE-TPGS | 5 | 50 | 5 | 2113.7 | 2122.7 | 2089.3 | 2077.5 |
| Tween 80 | 5 | 50 | 5 | 899.3 | 893.1 | 888.5 | 886.6 |
| Poloxamer 407 | 5 | 50 | 5 | 838.4 | 860.7 | 842.0 | 796.7 |
| Poloxamer 188 | 5 | 50 | 5 | 115.6 | 123.3 | 104.0 | 93.9 |
| Solutol HS15 | 5 | 50 | 5 | 1465.9 | 1501.6 | 1480.7 | 1456.3 |

| | | | | | | | |
|---|---|---|---|---|---|---|---|
| In the above table, "-" indicates that no surfactant was used. | | | | | | | |

The results demonstrated that surfactants SLS, Cremophor EL, Cremophor RH40, VE-TPGS, Tween 80, Poloxamer 407, and Solutol HS15 all significantly improved the solubility of compound 1.

### Example 3: Preparation of solid dispersion (SDD) of compound 1 by solvent method

According to the carrier polymer, solvent composition, and material ratio shown in Table 2 below, 9 mg of compound 1 and 21 mg of the carrier polymer (a mass ratio of 3:7) were weighed and transferred to a 40 mL glass vial, respectively, followed by addition of 1 mL of the corresponding solvent to prepare a sample solution with a drug loading of 30%. All sample solutions were transferred to an oven at 80°C and left until the solvent completely evaporated. The appearance of the resulting solids was observed and recorded.

**Table 2: Preparation of solid dispersions of compound 1 by solvent method**

| Sample | Solvent (volume ratio) | Appearance | | XRPD |
|---|---|---|---|---|
| | | Appearance of solution | After rapid drying | |
| API: PVP-K30 = 3:7 | Acetone: methanol = 9:1 | | | Amorphous |
| API: PVP-VA64 = 3:7 | Acetone: dichloromethane = 9:1 | | | |
| API: Soluplus = 3:7 | Acetone: dichloromethane = 9:1 | | | |
| API: HPMC E3 = 3:7 | Acetone: methanol = 9:1 | | | |
| API: HPMCASMG = 3:7 | Acetone: dichloromethane = 9:1 | Clear | Off-white film | Weak diffraction peak |
| API: HPMCASHG = 3:7 | Acetone: dichloromethane = 9:1 | | | Amorphous |
| API: HPCSSL = 3:7 | Acetone: dichloromethane = 9:1 | | | |
| API: Eudragit L100 = 3:7 | Acetone: methanol = 9:1 | | | Weak diffraction peak |

### Example 4: Preparation of solid dispersions of compound 1 by spray drying method

Solid dispersions corresponding to compound 1 were prepared by spray drying using the carrier polymers and solvents shown in Table 3 below.

Compound 1 and the corresponding carrier polymer were added to the corresponding solvent at a drug loading of 30%, stirred at 700 rpm until completely dissolved, followed by spray drying. All spray-dried samples were vacuum-dried at 50°C for 20 to 24 hours and characterized by polarized light microscopy (PLM), X-ray powder diffraction (XRPD), differential scanning calorimetry (DSC), dynamic vapor sorption (DVS), and thermogravimetric analysis (TGA), respectively. The formulation and spray drying parameters for each solid dispersion are shown in Table 3, and the characterization results of PLM, XRPD, DVS, DSC, and TGA are shown in Table 4.

**Table 3: Material ratios and preparation parameters for solid dispersions prepared by spray drying method**

| **Formulation No.** | **Formulation 1** | **Formulation 2** | **Formulation 3** | **Formulation 4** | **Formulation 5** | **Formulation 6** | **Formulation 7** | **Formulation 8** | **Formulation 9** |
|---|---|---|---|---|---|---|---|---|---|
| Formulation ratio | API: PVP-VA64 (3:7, *w*/*w*) | API: PVP-K30 (3:7, *w*/*w*) | API: HPMC ASMG (3:7, *w*/*w*) | API: PVP-VA64: Cremophor RH40 (30:65:5, *w*/*w*) | API: PVP-VA64: SLS (30:65:5 , *w*/*w*) | API: PVP-VA64: VE-TPGS (30:65:5 , *w*/*w*) | API: PVP-K30: Cremophor RH40 (30:65:5, *w*/*w*) | API: PVP-K30: SLS (30:65:5 , *w*/*w*) | API: PVP-K30: VE-TPGS (30:65:5 , *w*/*w*) |
| Solvent (volume ratio) | Acetone : DCM = 6:4 | Acetone : water = 7:3 | Acetone | Acetone: DCM = 6:4 | Acetone : EtOH = 1:1 | Acetone : DCM = 6:4 | Acetone: water = 7:3 | Acetone : water = 7:3 | Acetone : water = 7:3 |
| Conc. of API (mg/mL) | 20 | 20 | 20 | 20 | 15 | 20 | 20 | 20 | 20 |
| Amount of API | 1 g | 1 g | 1 g | 4 g | 4 g | 4 g | 6 g | 4 g | 4 g |
| Amount of carrier | 2.33 g | 2.33 g | 2.33 g | 8.67 g | 8.67 g | 8.67 g | 13 g | 8.67 g | 8.67 g |
| Amount of surfactant | - | - | - | 0.67 g | 0.67 g | 0.67 g | 1 g | 0.67 g | 0.67 g |
| Pump speed% | 35 | 25 | 35 | 35 | 35 | 35 | 25 | 25 | 25 |
| Pump power% | 100 | | | | | | | | |
| Nitrogen (mm) | 35 | | | | | | | | |
| Actual inlet | 84 to 86 | 99 to 106 | 89 to 92 | 84 to 87 | 83 to 85 | 84 to 86 | 100 | 100 to 103 | 100 |
| temp. (°C) | | | | | | | | | |
| Outlet temp. (°C) | 47 to 49 | 46 to 56 | 47 to 49 | 49 to 51 | 46 to 48 | 51 to 53 | 50 to 53 | 50 to 51 | 50 to 51 |

| | | | | | | | | | |
|---|---|---|---|---|---|---|---|---|---|
| In the above table, "-" indicates that no surfactant was used. | | | | | | | | | |

**Table 4: Summary of characterization results for solid dispersions prepared by spray drying method**

| **Formulation No.** | | **Formulation 1** | **Formulation 2** | **Formulation 3** | **Formulation 4** | **Formulation 5** | **Formulation 6** | **Formulation 7** | **Formulation 8** | **Formulation 9** |
|---|---|---|---|---|---|---|---|---|---|---|
| Appearance | | White powder | | | | | | | | |
| PLM | | Irregular block & no birefringence | | | | | | | | |
| XRPD | | Amorphous | | | | | | | | |
| Tg (°C)/m DSC | | 118.55 | 158.13 | 93.38 | 110 | 109 | 112 | 149 | 148 | 155 |
| TGA (%) | | 2.69 | 6.24 | 1.7 | 3.6 | 3.7 | 3.6 | 7.6 | 7.2 | 7.6 |
| HP LC | Drug loading (%) | 29.4 | 29.5 | 32.0 | 29.4 | 29.7 | 29.4 | 30.2 | 29.9 | 30.6 |
| | Purity (%) | 99.9 | 99.9 | 99.9 | 99.9 | 99.9 | 99.9 | 99.9 | 99.9 | 99.9 |
| | Content (%) | 98.0 | 98.2 | 107.0 | 100.0 | 100.0 | 100.1 | 100.0 | 100.1 | 100.1 |
| GC | Acetone | 120 ppm | 520 ppm | 61 ppm | 41 ppm | 77 ppm | 41 ppm | 1007 ppm | 27.43 ppm | 109.56 ppm |
| | DCM | ND | N/A | 9 ppm | 9 ppm | N/A | 9 ppm | N/A | N/A | N/A |
| | Ethanol | N/A | N/A | N/A | N/A | 31 ppm | N/A | N/A | N/A | N/A |
| Yield/% | | 80.0 | 80.0 | 76.0 | 80 | 85 | 78 | 60 | 72 | 60 |

The characterization results demonstrated that the prepared samples were all amorphous, and the residual solvents were in compliance with the requirements of the pharmacopoeia.

### Example 5: Dynamic solubility test of solid dispersions

20 mg each of the solid dispersion products from formulations 1 to 9 in Example 4 was taken and added with 3 mL of SGF (target concentration of API: 2 mg/mL). The mixture was stirred at 37°C and 500 rpm for 0.25 hours and 0.5 hours, respectively, followed by sampling. 3 mL of 2× FaSSIF was then added to adjust the solution pH to approximately 6.5. The mixture was stirred for another 0.25 hours, 0.5 hours, 1 hour, and 1.5 hours, respectively, followed by sampling. The collected samples were analyzed by UPLC, and the final solution pH was measured. The test results are shown in Table 5.

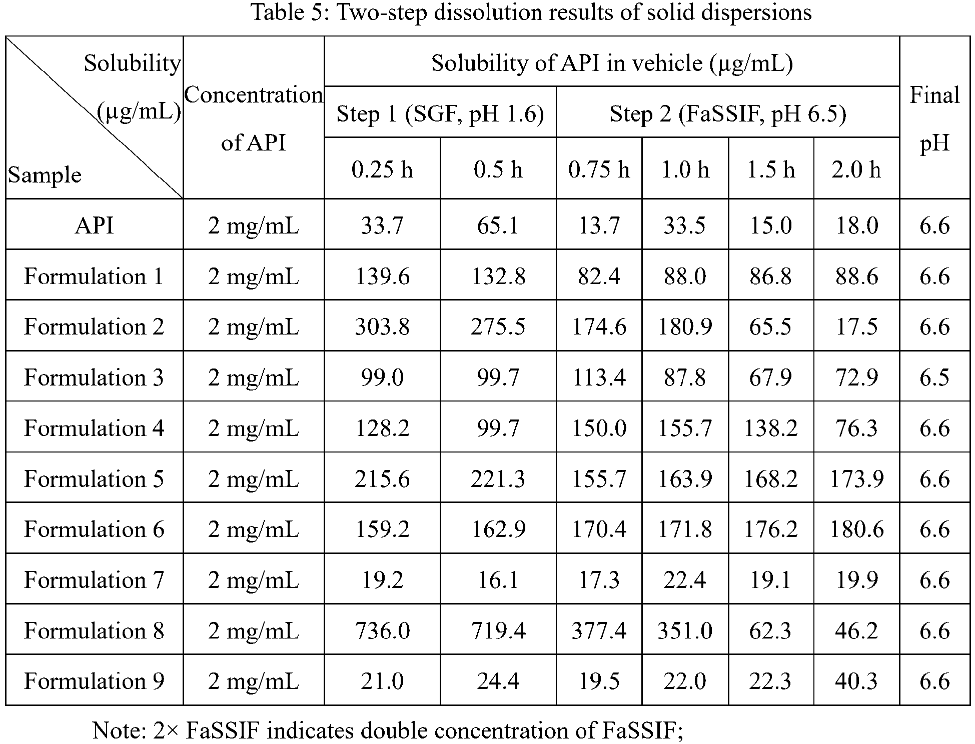

The results demonstrated that the solid dispersions using PVP-K30, PVP-VA64, and HPMCASMG as carriers all exhibited significantly improved dissolution compared to the API. Moreover, the dissolution data for the solid dispersions from formulations 5 and 6 using PVP-VA64 as the carrier indicated that the solubility changes of the solid dispersions from formulations 5 and 6 remained stable over time and were within an appropriate range.

### Example 6: Stability test of solid dispersions of compound 1 prepared by spray drying method

The stability of the solid dispersion products prepared according to Example 3 and Example 4 was evaluated under conditions of 25°C/60% RH (open) and 40°C/75% RH (open), respectively. Samples were taken at 2 weeks and 4 weeks, and tested for appearance, purity, and impurities. The results are shown in Tables 6 to 7.

**Table 6: Stability results of solid dispersions of compound 1 prepared by spray drying at 2 and 4 weeks**

| **Sample** | **Condition** | | **Time point** | **Total impurities (%)** | **Purity (%)** | **Content (%)** | **Relative retention time of single impurity** | |
|---|---|---|---|---|---|---|---|---|
| | | | | | | | **0.81** | **1** |
| **Formulation 1** | - | | Initial | 0.12 | 99.88 | 98 | 0.12 | 99.88 |
| | 25°C/60% RH | Open | 2 weeks | 0.11 | 99.89 | 97.2 | 0.11 | 99.89 |
| | | | 4 weeks | 0.1 | 99.91 | 99.5 | 0.1 | 99.91 |
| | 40°C/75% RH | | 2 weeks | 0.11 | 99.89 | 99.4 | 0.11 | 99.89 |
| | | | 4 weeks | 0.1 | 99.91 | 98.4 | 0.1 | 99.91 |
| **Formulation 2** | - | | Initial | 0.11 | 99.89 | 98.2 | 0.11 | 99.89 |
| | 25°C/60% RH | Open | 2 weeks | 0.1 | 99.9 | 99.8 | 0.1 | 99.9 |
| | | | 4 weeks | 0.09 | 99.91 | 99.3 | 0.09 | 99.91 |
| | 40°C/75% RH | | 2 weeks | 0.12 | 99.88 | 99.5 | 0.12 | 99.88 |
| | | | 4 weeks | 0.1 | 99.9 | 98 | 0.1 | 99.9 |
| **Formulation 3** | - | | Initial | 0.11 | 99.89 | 106.9 | 0.11 | 99.89 |
| | 25°C/60% RH | Open | 2 weeks | 0.12 | 99.88 | 101.9 | 0.12 | 99.88 |
| | | | 4 weeks | 0.09 | 99.91 | 100.6 | 0.09 | 99.91 |
| | 40°C/75% RH | | 2 weeks | 0.12 | 99.88 | 102.1 | 0.12 | 99.88 |
| | | | 4 weeks | 0.15 | 99.85 | 100.2 | 0.09 | 99.85 |
| **Formulation 4** | - | | Initial | 0.11 | 99.9 | 100.00% | 0.11 | 99.9 |
| | 25°C/60% RH | Open | 2 weeks | 0.1 | 99.9 | 100.10% | 0.1 | 99.9 |
| | | | 4 weeks | 0.09 | 99.91 | 99.00% | 0.1 | 99.91 |
| | 40°C/75% RH | | 2 weeks | 0.09 | 99.91 | 99.60% | 0.09 | 99.91 |
| | | | 4 weeks | 0.09 | 99.91 | 99.60% | 0.09 | 99.91 |
| **Formulation 5** | - | | Initial | 0.12 | 99.88 | 100.00% | 0.12 | 99.88 |
| | 25°C/60% RH | Open | 2 weeks | 0.09 | 99.91 | 102.10% | 0.1 | 99.91 |
| | | | 4 weeks | 0.09 | 99.91 | 99.90% | 0.09 | 99.91 |
| | 40°C/75% RH | | 2 weeks | 0.1 | 99.9 | 102.20% | 0.1 | 99.9 |
| | | | 4 weeks | 0.1 | 99.9 | 99.40% | 0.1 | 99.9 |
| **Formulation 6** | - | | Initial | 0.12 | 99.88 | 100.10% | 0.12 | 99.88 |
| | 25°C/60% RH | Open | 2 weeks | 0.09 | 99.91 | 101.50% | 0.09 | 99.91 |
| | | | 4 weeks | 0.09 | 99.91 | 98.40% | 0.09 | 99.91 |
| | 40°C/75% RH | | 2 weeks | 0.09 | 99.91 | 99.00% | 0.1 | 99.91 |
| | | | 4 weeks | 0.09 | 99.91 | 98.90% | 0.09 | 99.91 |
| **Formulation 7** | - | | Initial | 0.09 | 99.91 | 100.00% | 0.1 | 99.91 |
| | 25°C/60% RH | Open | 2 weeks | 0.09 | 99.91 | 103.10% | 0.1 | 99.91 |
| | | | 4 weeks | 0.09 | 99.91 | 100.70% | 0.09 | 99.91 |
| | 40°C/75% RH | | 2 weeks | 0.1 | 99.9 | 103.00% | 0.1 | 99.9 |
| | | | 4 weeks | 0.1 | 99.9 | 101.00% | 0.1 | 99.9 |
| **Formulation 8** | - | | Initial | 0.09 | 99.91 | 100.10% | 0.09 | 99.91 |
| | 25°C/60% RH | Open | 2 weeks | 0.09 | 99.91 | 101.30% | 0.1 | 99.91 |
| | | | 4 weeks | 0.09 | 99.91 | 100.90% | 0.09 | 99.91 |
| | 40°C/75% RH | | 2 weeks | 0.1 | 99.9 | 101.60% | 0.1 | 99.9 |
| | | | 4 weeks | 0.1 | 99.9 | 101.30% | 0.1 | 99.9 |
| **Formulation 9** | - | | Initial | 0.11 | 99.89 | 100.10% | 0.11 | 99.89 |
| | 25°C/60% RH | Open | 2 weeks | 0.09 | 99.91 | 102.80% | 0.09 | 99.91 |
| | | | 4 weeks | 0.09 | 99.91 | 101.70% | 0.09 | 99.91 |
| | 40°C/75% RH | | 2 weeks | 0.09 | 99.91 | 103.50% | 0.1 | 99.91 |
| | | | 4 weeks | 0.1 | 99.9 | 103.30% | 0.1 | 99.9 |

**Table 7: Physical stability results of solid dispersions of compound 1 prepared by spray drying**

| Product | Test item | Initial | 25°C/60% RH (open) | | 40°C/75% RH (open) | |
|---|---|---|---|---|---|---|
| | | | 2 weeks | 4 weeks | 2 weeks | 4 weeks |
| Formulation 1 | XRPD | Amorphous | Amorphous | Amorphous | Amorphous | Amorphous |
| | Appearance | White powder | Slight caking | Slight caking | Caking | Caking |
| Formulation 2 | XRPD | Amorphous | Amorphous | Amorphous | Amorphous | Amorphous |
| | Appearance | White powder | Slight caking | Slight caking | Caking | Caking |
| Formulation 3 | XRPD | Amorphous | Amorphous | Amorphous | Amorphous | Amorphous |
| | Appearance | White powder | Slight caking | Slight caking | Caking | Caking |
| Formulation 4 | XRPD | Amorphous | Amorphous | Amorphous | Amorphous | Amorphous |
| | Appearance | White powder | Slight caking | Slight caking | Caking | Caking |
| Formulation 5 | XRPD | Amorphous | Amorphous | Amorphous | Amorphous | Amorphous |
| | Appearance | White powder | Slight caking | Slight caking | Caking | Caking |
| Formulation 6 | XRPD | Amorphous | Amorphous | Amorphous | Amorphous | Amorphous |
| | Appearance | White powder | Slight caking | Slight caking | Caking | Caking |
| Formulation 8 | XRPD | Amorphous | Amorphous | Amorphous | Amorphous | Amorphous |
| | Appearance | White powder | Slight caking | Slight caking | Caking | Caking |

The results demonstrated that all samples exhibited good chemical stability under 25°C/60% RH (open) and 40°C/75% RH (open) conditions, with no significant increase in impurities.

The physical stability results indicated that the samples from formulations 1 to 6 and formulation 8 showed no significant changes in appearance except for partial caking.

The solid dispersions from formulations 4, 5, 6, and 8 exhibited good physical stability after 4 weeks of storage under 25°C/60% RH (open) and 40°C/75% RH (open) conditions, and all were amorphous.

### Example 7: Preparation of scale-up solid dispersion samples

A spray drying solution with a concentration of 15 mg/mL (calculated based on API) was prepared using a mixture of acetone: ethanol: water = 4:3.5:2.5 (v:v:v) as the solvent. Scale-up sample preparation was carried out according to the proposed process parameters (see Table 8).

50 g of compound 1 was weighed according to the formulation list and added to acetone, stirred until completely dissolved, followed by addition of ethanol and water with thorough mixing. PVP VA64 and SLS in the formulation amount were then added to the solvent. All solids were stirred at 700 rpm until completely dissolved, followed by spray drying. The spray-dried samples were vacuum-dried at 50°C for 20 to 24 hours to obtain the product.

**Table 8: Material ratios and preparation parameters for scale-up samples**

| | |
|---|---|
| Formulation ratio (wt.%) | API: PVP VA64: SLS (30:65:5, w/w/w) |
| Target drug loading (%, w/w) | 30% |
| Concentration of API (mg/mL) | 15 |
| API batch | 50 g |
| Solvent (volume ratio) | Acetone: ethanol: water = 4:3.5:2.5 (v/v/v) |
| Appearance of solution | Clear and transparent |
| Actual inlet temperature (°C) | 99 to 101 |
| Actual outlet temperature (°C) | 61 to 63 |
| Pump speed/% | 30 |

### Example 8: Preparation of solid dosage forms and disintegration test

Preparation Examples 1 to 5 and Comparative Examples 1 to 7 in Table 9 below were all prepared by a dry granulation process. The specific procedure was as follows. Materials were accurately weighed according to the formulation, wherein the solid dispersion of compound 1 was not sieved, while the other excipients were sieved for later use. The intra-granular filler, disintegrant, glidant, and solid dispersion of compound 1 were pre-mixed, followed by milling and sieving. A lubricant was added for dry granulation. The extra-granular filler, disintegrant, swelling agent, and lubricant were added for final blending. After final blending, the mixture was packaged according to the required amount of the active ingredient, and then compressed into tablets of pharmaceutical specifications.

The obtained tablets were subjected to a disintegration test in accordance with the disintegration time limit in the Chinese Pharmacopoeia 2020 Edition. The disintegration times for each preparation example and comparative example are shown in Table 9 and Table 10 below.

**Table 9: Material ratios and disintegration test results of preparation examples of solid dosage forms**

| Material name | Preparation Example 1 | Preparation Example 2 | Preparation Example 3 | Preparation Example 4 | Preparation Example 5 |
|---|---|---|---|---|---|
| | Amount (mg) | Amount (mg) | Amount (mg) | Amount (mg) | Amount (mg) |
| SDD of compound 1 | 333.36 | 333.36 | 333.36 | 333.36 | 333.36 |
| Microcrystalline cellulose PH101 | 80 | 80 | 80 | 80 | 80 |
| Mannitol 100SD | 34.64 | 34.64 | 34.64 | 34.64 | 34.64 |
| Croscarmellose sodium SD711 | 40 | 40 | 40 | 40 | 40 |
| Colloidal silicon dioxide | 8 | 8 | 8 | 8 | 8 |
| Magnesium stearate | 4 | 4 | 4 | 4 | 4 |
| Sodium chloride | - | - | - | 40 | - |
| Microcrystalline cellulose PH101 | 248 | 216 | 176 | 176 | 216 |
| Croscarmellose sodium SD711 | 40 | 40 | 40 | 40 | 40 |
| Magnesium stearate | 4 | 4 | 4 | 4 | 4 |
| Sodium bicarbonate | - | - | - | - | 40 |
| Sodium chloride | 8 | 40 | 80 | 40 | - |
| Total weight of tablet (mg) | 800 | 800 | 800 | 800 | 800 |
| Average hardness (kp) | 20 | 20 | 20 | 20 | 20 |
| Disintegration time | 5' to 6'10" | 1'30" to 2' | 1'10" to 1'31" | 1'24" to 1'38" | 4'52" to 5'10" |
| Disintegration phenomenon | Tablets immediately absorb water, swell, and disintegrate into agglomerates and granules when dropped into water | Tablets immediately absorb water, swell, and disintegrate into agglomerates and granules when dropped into water | Tablets immediately absorb water, swell, and disintegrate into agglomerates and granules when dropped into water | Tablets immediately absorb water, swell, and disintegrate into agglomerates and granules when dropped into water | Tablets immediately absorb water, swell, and disintegrate into agglomerates and granules when dropped into water |

**Table 10: Material ratios and disintegration test results of comparative examples of solid dosage forms**

| Material name | Comp. Ex. 1 | Comp. Ex. 2 | Comp. Ex. 3 | Comp. Ex. 4 | Comp. Ex. 5 | Comp. Ex. 6 | Comp. Ex. 7 |
|---|---|---|---|---|---|---|---|
| | Amount (mg) | Amount (mg) | Amount (mg) | Amount (mg) | Amount (mg) | Amount (mg) | Amount (mg) |
| Intra-granular materials | | | | | | | |
| Solid dispersion of compound 1 | 333.5 | 333.5 | 333.5 | 333.5 | 332.8 | 332.8 | 333.36 |
| Microcrystalline cellulose PH101 | 81.5 | 81.5 | 81.5 | 81.5 | 59.2 | 59.2 | 80 |
| Mannitol 100SD | 50 | 50 | 50 | 50 | / | / | 34.64 |
| Croscarmellose sodium SD711 | 12.5 | 12.5 | 12.5 | 12.5 | 40 | 40 | 40 |
| Anhydrous dicalcium phosphate | / | / | / | / | 56 | 304 | / |
| Colloidal silicon dioxide | 5 | 5 | 5 | 5 | / | / | 8 |
| Hydrophobic silicon dioxide | / | / | / | / | 8 | 8 | / |
| Magnesium stearate | 2.5 | 2.5 | 2.5 | 2.5 | 4 | 4 | 4 |
| Sodium chloride | / | / | / | / | / | / | / |

| Extra-granular materials | | | | | | | |
|---|---|---|---|---|---|---|---|
| Microcrystalline cellulose PH101 | 285 | 245 | / | / | / | / | 256 |
| Mannitol M100 | / | / | 252 | / | / | / | / |
| Anhydrous dicalcium phosphate | / | / | / | 252 | 248 | / | / |
| Croscarmellose sodium SD711 | 27.5 | 67.5 | 12.5 | 12.5 | 40 | 40 | 40 |
| Crospovidone | / | / | 40 | 40 | / | / | / |
| Colloidal silicon dioxide | / | / | 8 | 8 | / | / | / |
| Hydrophobic silicon dioxide | / | / | / | / | 8 | 8 | / |
| Magnesium stearate | 2.5 | 2.5 | 2.5 | 2.5 | 4 | 4 | 4 |
| Total weight of tablet | 800 | 800 | 800 | 800 | 800 | 800 | 800 |

| Test item | | | | | | | |
|---|---|---|---|---|---|---|---|
| Hardness (kp) | 20 | 20 | 20 | 20 | 10 | 10 | 20 |
| Disintegration time | 16'35" to 18'17" | 12'28" to 13'45" | 16'40" to 17'46" | 20'7" to 21'7" | ~22 min | ~23 min | 18' to 18'45" |
| Disintegration phenomenon | Erosion | Erosion | Erosion | Erosion | Erosion; tablets disintegrate layer by layer from the surface | Erosion; tablets disintegrate layer by layer from the surface | Erosion |

| | | | | | | | |
|---|---|---|---|---|---|---|---|
| Note: "/" in Tables 9 and 10 indicates that the component was not included. | | | | | | | |

The disintegration time results demonstrated that:
The tablets in Comparative Example 7 without inorganic salts as well as Comparative Examples 1 and 3 to 6 all exhibited disintegration times exceeding the pharmacopoeial limits. The disintegration time of the tablets in Preparation Example 1 with the addition of 1% sodium chloride was shortened to approximately 6 minutes. The disintegration time of the tablets in Preparation Example 2 with the addition of 5% sodium chloride was shortened to approximately 2 minutes. The disintegration time of the tablets in Preparation Example 3 with the addition of 10% sodium chloride was shortened to 1.5 minutes. The disintegration time of the tablets in Preparation Example 4 with 5% sodium chloride added intra-granularly and 5% sodium chloride added extra-granularly was shortened to 1.5 minutes. The disintegration time of the tablets in Preparation Example 5 with the addition of 5% sodium bicarbonate was shortened to approximately 5 minutes. Thus, the disintegration times of the tablets in Preparation Examples 1 to 5 were significantly improved and all shortened to within the pharmacopoeial limits. Moreover, the tablets in Comparative Examples 1 to 7 was disintegrated by erosion, whereas the tablets in Preparation Examples 1 to 5 was disintegrated by water absorption, swelling, and disintegration into agglomerates or granules.

These findings indicated that the addition of inorganic salts can effectively inhibit the gelation of PVP-VA64, thereby promoting rapid tablet disintegration. Sodium chloride exhibited the most pronounced disintegration-promoting effect, reducing the disintegration time to 6 minutes with only 1% of the formulation amount and to within 2 minutes with an addition of 5% and 10%. Sodium bicarbonate also exhibited a good disintegration-promoting effect, reducing the disintegration time to 5 minutes with an addition of 5%.

### Example 9: Dissolution test of solid dosage forms

Based on the pH values of commonly used dissolution media and the solubility results of the aforementioned solid dispersions, pH 1.2 hydrochloric acid medium and pH 1.2 hydrochloric acid + 0.3% SLS medium were selected to determine the dissolution. The dissolution test methods are shown in Tables 11 and 12, and the analytical data are shown in Tables 13 and 14.

**Table 11: Dissolution test method for solid dosage forms**

| | |
|---|---|
| Dissolution apparatus | USP <711> Apparatus 2 (paddle method) |
| Rotation speed | 75 rpm |
| Dissolution medium | pH 1.2 hydrochloric acid medium, pH 1.2 hydrochloric acid + 0.3% SLS medium |
| Medium volume | 900 mL |
| Medium temperature | 37 ± 0.5°C |
| Sampling method | Automatic or manual |
| Sampling time points | 5, 10, 15, 20, 30, 45, 60 min and 30 min at the maximum speed of 250 rpm |
| Sample volume | 5 mL (manual), 1.5 mL (automatic) |
| Waste discard volume | 0.5 mL (automated only) |
| Primary filtration | 10 µm full-flow filter |
| Filtration | 0.45 µm RC filter membrane, discard the first 3 mL and collect the filtrate (manual) |

**Table 12: Liquid chromatography parameters of dissolution test method for solid dosage forms**

| | | | |
|---|---|---|---|
| Mobile phase A | 0.03% trifluoroacetic acid in purified water (v/v) | | |
| Mobile phase B | 0.03% trifluoroacetic acid in acetonitrile (v/v) | | |
| Chromatographic column | Agilent SB-C8, 20 mm × 4.6 mm, 1.8 µm or equivalent | | |
| Detection wavelength | 245 nm | | |
| Injection volume | 5 µL | | |
| Column temperature | 40°C | | |
| Autosampler temperature | 5°C | | |
| Flow rate | 1.0 mL/min | | |
| Isocratic | Time | A% | B% |
| | 0.0 | 65 | 35 |
| | 3.0 | 65 | 35 |
| Run time | 3.0 min | | |
| Needle wash solution | Acetonitrile: purified water = 1:1 (v/v) | | |

**Table 13: Dissolution data of solid dosage forms of Comparative Examples 2, 4, 7 and Preparation Examples 1 to 5 in pH 1.2 hydrochloric acid medium**

| Group | Time point (min) | Dissolution% (paddle method, 75 rpm, pH 1.2 hydrochloric acid, 900 mL) | | | | | | | | |
|---|---|---|---|---|---|---|---|---|---|---|
| | | 0 | 5 | 10 | 15 | 20 | 30 | 45 | 60 | 90 |
| Comparative Example 2 | Avg. Dis | 0 | 10 | 15 | 19 | 22 | 27 | 32 | 36 | 46 |
| | RSD% | N/A | 2.6 | 1.1 | 2.5 | 0.7 | 3.0 | 0.3 | 0.5 | 0.6 |
| Comparative Example 4 | Avg. Dis | 0 | 4 | 8 | 11 | 13 | 18 | 23 | 26 | 38 |
| | RSD% | N/A | 7.6 | 4.0 | 3.2 | 5.2 | 1.9 | 1.7 | 1.3 | 0.1 |
| Comparative Example 7 | Avg. Dis | 0 | 1 | 5 | 11 | 16 | 23 | 29 | 34 | 46 |
| | RSD% | NA | 0 | 0 | 6.7 | 4.6 | 3.1 | 0 | 0 | 0 |
| Preparation Example 1 | Avg. Dis | 0 | 4 | 8 | 13 | 17 | 22 | 28 | 33 | 47 |
| | RSD% | NA | 20.2 | 0 | 0 | 4.3 | 6.4 | 5.1 | 4.3 | 0 |
| Preparation Example 2 | Avg. Dis | 0 | 3 | 8 | 12 | 15 | 19 | 25 | 29 | 47 |
| | RSD% | NA | 0 | 9.4 | 6.1 | 4.9 | 3.8 | 2.9 | 2.5 | 0 |
| Preparation Example 3 | Avg. Dis | 0 | 7 | 15 | 19 | 24 | 29 | 35 | 40 | 50 |
| | RSD% | NA | 0 | 4.9 | 7.4 | 3 | 4.9 | 2 | 1.8 | 1.4 |
| Preparation Example 4 | Avg. Dis | 0 | 9 | 15 | 21 | 25 | 31 | 37 | 41 | 51 |
| | RSD% | NA | 8.3 | 9.4 | 6.7 | 5.7 | 4.6 | 5.8 | 5.2 | 1.4 |
| Preparation Example 5 | Avg. Dis | 0 | 5 | 15 | 22 | 27 | 33 | 39 | 44 | 50 |
| | RSD% | NA | 0 | 0 | 0 | 0 | 0 | 0 | 1.6 | 1.4 |

**Table 14: Dissolution data of solid dosage forms of Comparative Example 7 and Preparation Examples 1 to 5 in pH 1.2 hydrochloric acid + 0.3% SLS medium**

| Group | | Dissolution% | | | | | | | | |
|---|---|---|---|---|---|---|---|---|---|---|
| | Time point (min) | (paddle method, 75 rpm, pH 1.2 hydrochloric acid + 0.3% SLS, 900 mL) | | | | | | | | |
| | | 0 | 5 | 10 | 15 | 20 | 30 | 45 | 60 | 90 |
| Comp. Ex. 7 | Avg. Dis | 0 | 10 | 33 | 48 | 58 | 72 | 84 | 91 | 98 |
| | RSD% | NA | 0 | 2.2 | 1.5 | 0 | 1 | 1.7 | 0 | 0 |
| Prep. Ex. 1 | Avg. Dis | 0 | 28 | 46 | 58 | 67 | 78 | 87 | 92 | 97 |
| | RSD% | NA | 5.1 | 1.6 | 2.4 | 1.1 | 1.8 | 0 | 0 | 0 |
| Prep. Ex. 2 | Avg. Dis | 0 | 23 | 38 | 47 | 54 | 63 | 72 | 79 | 95 |
| | RSD% | NA | 9.4 | 9.4 | 9 | 6.6 | 6.7 | 3.9 | 1.8 | 1.5 |
| Prep. Ex. 3 | Avg. Dis | 0 | 36 | 55 | 67 | 74 | 82 | 89 | 93 | 99 |
| | RSD% | NA | 6 | 2.6 | 1.1 | 1 | 0 | 0 | 0 | 0 |
| Prep. Ex. 4 | Avg. Dis | 0 | 35 | 55 | 66 | 73 | 82 | 88 | 92 | 96 |
| | RSD% | NA | 4 | 0 | 0 | 0 | 0 | 0 | 0.8 | 1.5 |
| Prep. Ex. 5 | Avg. Dis | 0 | 26 | 53 | 66 | 75 | 86 | 93 | 97 | 99 |
| | RSD% | NA | 2.8 | 0 | 0 | 0 | 0.8 | 0 | 0 | 0 |

The dissolution results demonstrated that:
In pH 1.2 hydrochloric acid medium, Preparation Examples 3 to 5 exhibited faster dissolution rates compared to Comparative Examples 2, 4, and 7.

In pH 1.2 hydrochloric acid + 0.3% SLS medium, Comparative Example 7 and Preparation Examples 1 to 5 achieved a final dissolution of approximately 100%. Preparation Examples 3 to 5 exhibited faster dissolution rates compared to Comparative Example 7 and Preparation Examples 1 to 2. Specifically, Preparation Example 5 reached a dissolution of 86% at 30 minutes, showing the most significant advantage. In summary, in terms of disintegration time, the tablets in Preparation Examples 1 to 5 can effectively shorten the disintegration time to within the pharmacopoeial limits. Based on the dissolution data and dissolution phenomena, Preparation Examples 1 and 5 exhibit faster dissolution rates and higher dissolution compared to Preparation Examples 2, 3, and 4, and rapidly disintegrated into granules in dissolution vessels without forming agglomerates.

### Example 10: Pharmacokinetic study

### Experimental Preparation 1

SPF-grade male SD rats weighing 160 to 320 g were divided into 2 groups, with 3 rats per group [Source: Beijing Vital River Laboratory Animal Technology Co., Ltd., Certificate No.: 1103242011040898].

Formulation and dosing: According to the drug concentration and dosing requirements in Table 15 below, the API was accurately weighed and added to the required volume of 5% Solutol. The mixture was vortexed for 2 minutes, then sonicated for 10 minutes, and stirred for 1 hour before administration.

**Table 15: Formulation and dosing regimen for API pharmacokinetic study**

| Test substance | Route of administration | Vehicle | Dose (mg/kg) | Drug concentration (mg/mL) | Dosing volume (mL/kg) |
|---|---|---|---|---|---|
| API | i.g. | 5% Solutol | 30 | 3 | 10 |
| API | i.g. | 5% Solutol | 300 | 30 | 10 |

### Experimental Preparation 2

SPF-grade male SD rats weighing 160 to 320 g were divided into 6 groups, with 3 rats per group [Source: Beijing Vital River Laboratory Animal Technology Co., Ltd., Certificate No.: 110011210113195883 (male)].

Formulation and dosing: 80 mg of the corresponding sample in the table below was accurately weighed, dispersed in the required volume of pure water, vortexed for 5 minutes, and administered intragastrically (i.g.). The experimental dosing was scaled proportionally based on animal body weight and dosing concentration.

### Sample collection and preparation

After administration to the SD rats in each group of Experimental Preparation 1 and Experimental Preparation 2, approximately 0.2 to 0.3 mL of blood samples were collected from the orbital venous plexus at 0.167, 0.5, 1, 2, 3, 4, 6, 8, 10, and 24 hours post-dosing, and transferred to an anticoagulant EP tube (containing 4 µL of EDTA-K2, 375 mg/mL), which was gently inverted three times, stored in an ice box (no more than 30 minutes), centrifuged at 3500 × g for 10 minutes at 4°C, and plasma supernatant was transferred to a labeled EP tube. Hemolysis was observed and recorded, followed by bioanalytical testing.

### Sample analysis

Sample pretreatment: 40 µL of sample was mixed with 160 µL of acetonitrile containing 0.1% FA and 200 ng/mL mixed internal standard to precipitate proteins. The mixture was vortexed thoroughly and centrifuged at 13,000 rpm for 10 minutes at 4°C.

110 µL of the supernatant was transferred to another 96-well deep-well plate, followed by addition of 110 µL of a mixture of methanol and water (1:3, v:v) containing 0.1% FA. The mixture was shaken for 10 minutes and analyzed using an LC-MS/MS system. The system employed a Sciex Exion LC AD high-performance liquid chromatography system with an XBridge BEH C18 (2.1 × 50 mm, 2.5 µm) column for gradient elution and an AB Sciex Qtrap 4500 mass spectrometer for analysis. The mass spectrometer employed an electrospray ionization (ESI) source in positive ion mode to monitor Tolbutamide, with precursor ion to product ion transitions with mass-to-charge ratios (m/z) of 415.1→263.1 and 271.1→155, respectively.

### Data analysis

Data were analyzed using WinNonlin (version 5.2.1, Pharsight, Mountain View, CA) with a non-compartmental model to obtain PK parameters (Cₘₐₓ, Tₘₐₓ, T_{1/2}, AUCₗₐₛₜ, *etc.*)*.*

**Table 15: Pharmacokinetic data of some solid dispersions**

| Sample | Route of administration | Vehicle | Animal | Dose (mg/kg) | Cₘₐₓ (ng/mL) | Tₘₐₓ (hr) | T_{1/2} (hr) | AUClast (ng/mL*hr) |
|---|---|---|---|---|---|---|---|---|
| Formulation 1 | i.g. | Water | Male SD rats | 30.00 | 3150 | 4.00 | 2.43 | 25316 |
| Formulation 2 | i.g. | Water | Male SD rats | 30.00 | 3697 | 0.67 | 2.91 | 16006 |
| Formulation 5 | i.g. | Water | Male SD rats | 30.00 | 2677 | 1.33 | 2.16 | 16746 |
| Formulation 8 | i.g. | Water | Male SD rats | 30.00 | 2790 | 4.00 | 2.30 | 20014 |
| Formulation 5 | i.g. | Water | Male SD rats | 300.00 | 18000 | 7.33 | 4.28 | 260251 |
| Formulation 8 | i.g. | Water | Male SD rats | 300.00 | 14967 | 1.50 | 3.42 | 90120 |

**Table 16: Pharmacokinetic data of API**

| Sample | Route of administration | Vehicle | Animal | Dose (mg/kg) | Cₘₐₓ (ng/mL) | Tₘₐₓ (hr) | T_{1/2} (hr) | AUClast (ng/mL*hr) |
|---|---|---|---|---|---|---|---|---|
| API | i.g. | 5% Solutol | Male SD rats | 30.00 | 2763 | 0.67 | 5.31 | 10814 |
| API | i.g. | 5% Solutol | Male SD rats | 300.00 | 2363 | 1 | 13.8 | 14681 |

The results demonstrated that after formulating compound 1 into solid dispersions, the obtained solid dispersions exhibited significant improvements in Cₘₐₓ, Tₘₐₓ, T_{1/2}, and AUCₗₐₛₜ values compared to the API, effectively enhancing the *in vivo* absorption and bioavailability of the API.

The embodiments of the present disclosure have been described above. However, the present disclosure is not limited to the aforementioned embodiments. Any modifications, equivalent substitutions, improvements, *etc.,* made within the spirit and principles of the present disclosure shall be included within the scope of protection of the present disclosure.

## Claims

1. A solid dispersion of a P2X4 inhibitor, comprising an active ingredient and a carrier; the active ingredient is at least one selected from the group consisting of compound 1 to compound 48 or a pharmaceutically acceptable salt thereof: 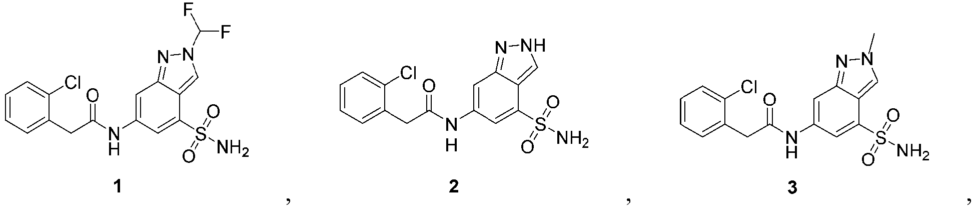 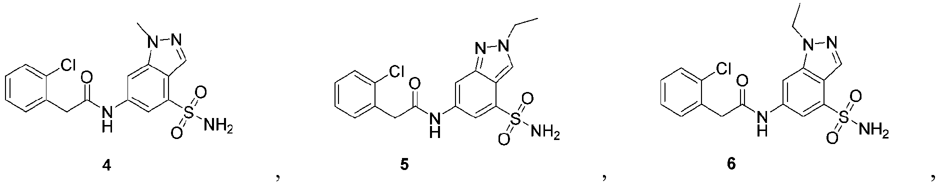 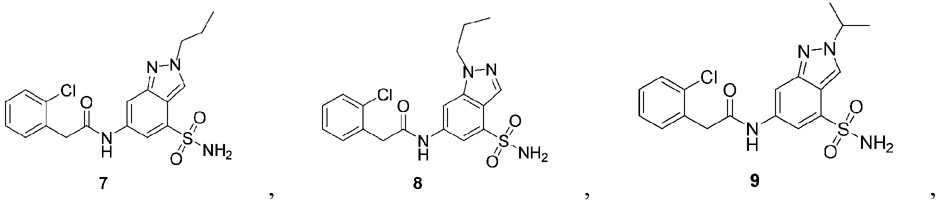 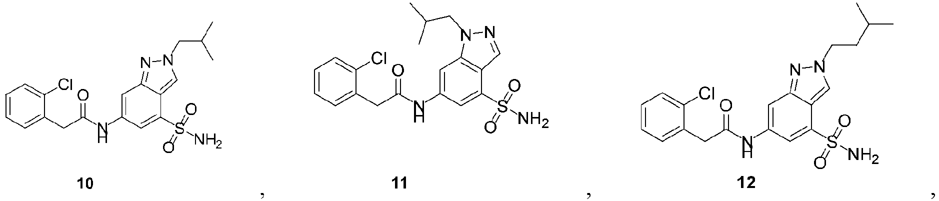 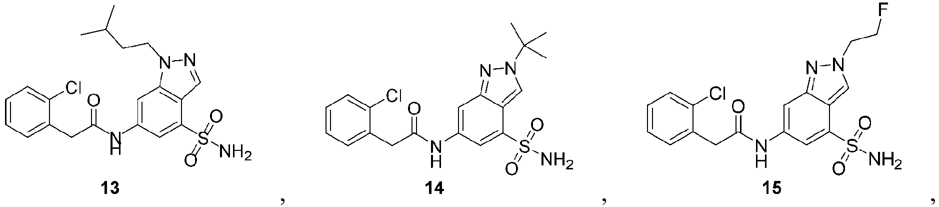 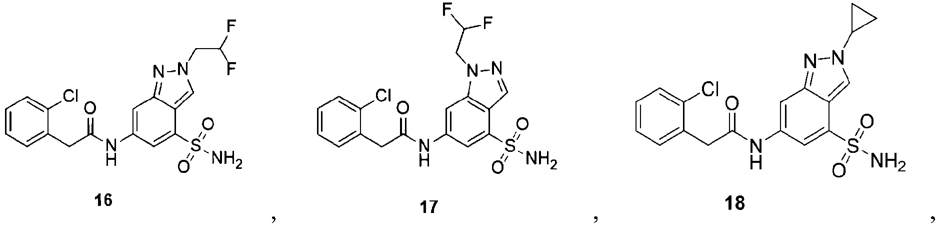 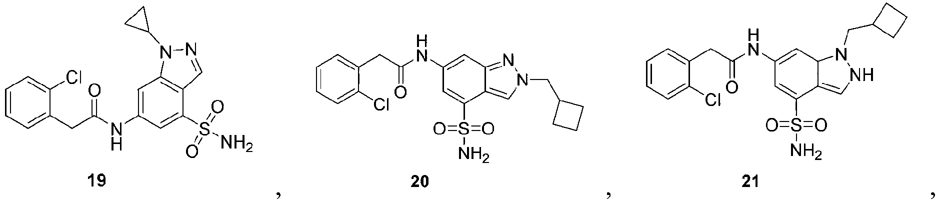 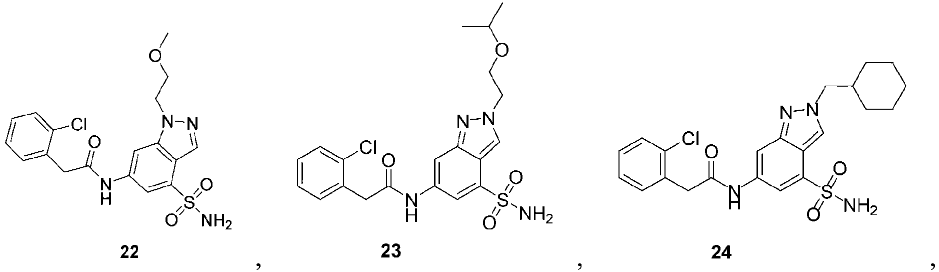 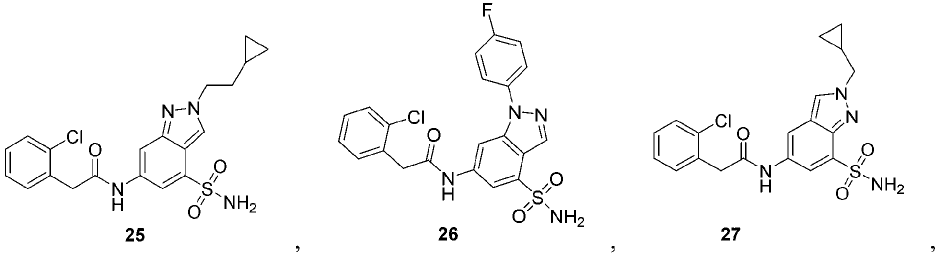 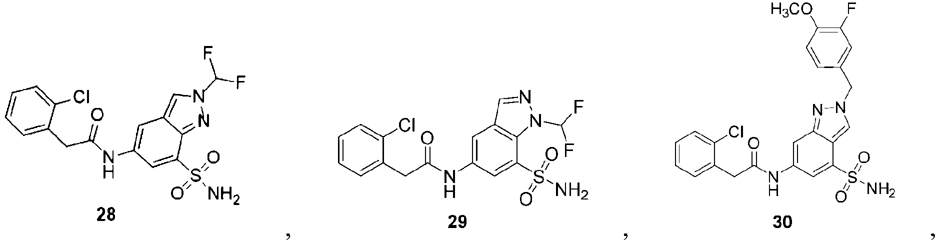 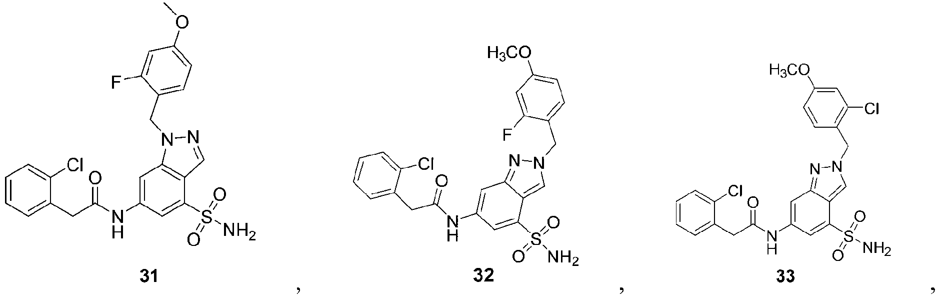 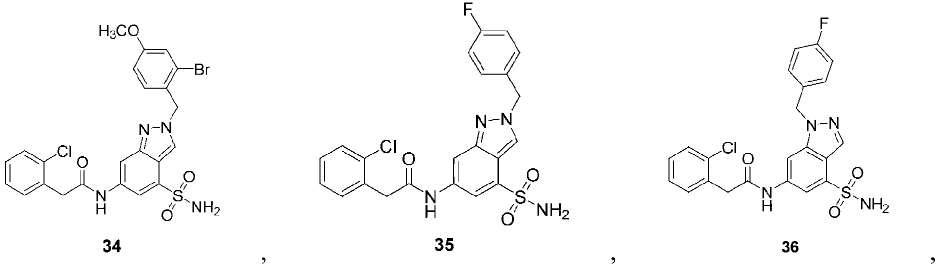 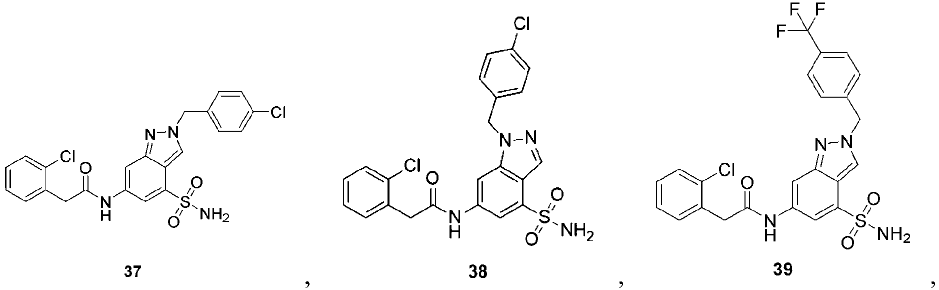 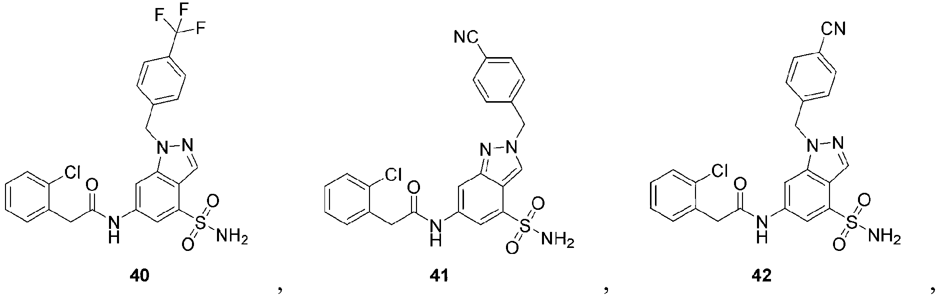 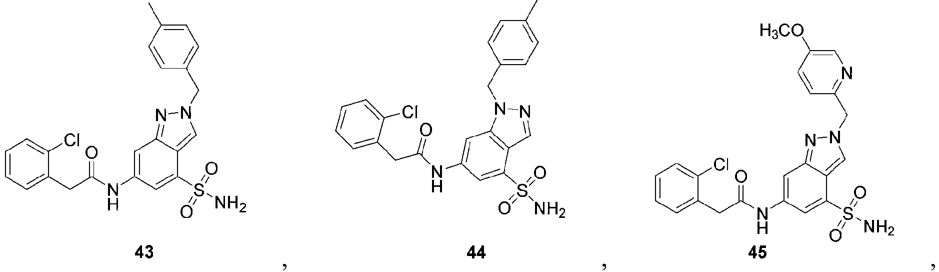 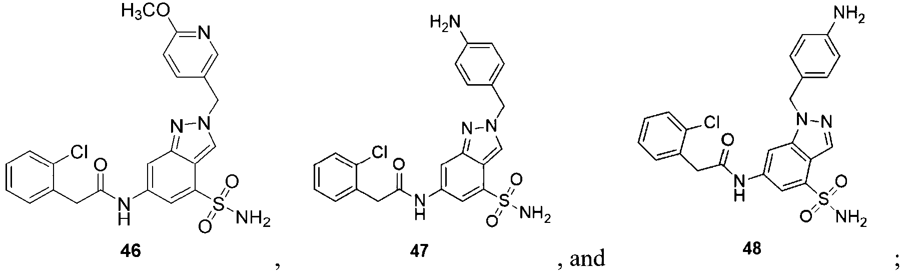 the carrier is one or more selected from the group consisting of copovidone, povidone, hypromellose acetate succinate, hypromellose phthalate, polyvinyl caprolactam-polyvinyl acetate-polyethylene glycol graft copolymer, hydroxypropyl cellulose, hypromellose, polyacrylic resin, cellulose acetate phthalate, methacrylic acid-methyl methacrylate copolymer, and polyethylene oxide.

2. The solid dispersion of the P2X4 inhibitor according to claim 1, wherein the solid dispersion further comprises a surfactant;
preferably, the surfactant is one selected from the group consisting of sodium lauryl sulfate, sodium cetyl sulfate, sodium stearyl sulfate, Tween, hydrogenated castor oil, glyceryl monostearate, vitamin E polyethylene glycol succinate, polyoxyethylene castor oil derivative, Poloxamer, and Solutol HS 15, or a mixture thereof obtained by mixing two or more thereof in any proportion;
more preferably, the surfactant is one selected from the group consisting of sodium lauryl sulfate, vitamin E polyethylene glycol succinate, and Cremophor RH40, or a mixture thereof obtained by mixing two or more thereof in any proportion.

3. The solid dispersion of the P2X4 inhibitor according to claim 2, wherein the active ingredient and the carrier have a mass ratio of 1:(1 to 7), preferably 1:(2 to 5);
preferably, the active ingredient, the carrier, and the surfactant have a mass ratio of 1:(1 to 7):(0.1 to 1), preferably 1:(2 to 5):(0.1 to 1).

4. The solid dispersion of the P2X4 inhibitor according to any one of claims 1 to 3, wherein the solid dispersion comprises: compound 1, copovidone, and Cremophor RH40;
or, compound 1, copovidone, and SLS;
or, compound 1, copovidone, and vitamin E polyethylene glycol succinate (VE-TPGS);
preferably, the solid dispersion comprises: compound 1, PVP-VA64, and SLS;
or, compound 1, PVP-VA64, and vitamin E polyethylene glycol succinate (VE-TPGS);
preferably, the solid dispersion comprises the following components in parts by weight: 1 part by weight of compound 1, 1 to 7 parts by weight of PVP-VA64, and 0.1 to 1 part by weight of SLS; or, 1 part by weight of compound 1, 1 to 7 parts by weight of PVP-VA64, and 0.1 to 1 part by weight of vitamin E polyethylene glycol succinate (VE-TPGS).

5. A preparation method for the solid dispersion as defined in any one of claims 1 to 4, wherein the solid dispersion is prepared using a spray drying method, a solvent method, or a hot-melt extrusion method, comprising the following steps:
spray drying method: adding a raw material to a solvent S1 to prepare a solution, followed by spray drying to obtain the solid dispersion;
solvent method: adding a raw material to a solvent S2 to prepare a solution, then removing the solvent from the resulting solution, followed by drying and pulverizing to obtain the solid dispersion;
hot-melt extrusion method: mixing a raw material, and optionally adding an appropriate amount of lubricant for hot-melt extrusion;
the solvent S1 is a ketone solvent, or a mixed solvent composed of a ketone solvent and a solvent selected from the group consisting of a halogenated alkane solvent, water, and an alcohol solvent;
the solvent S2 is a ketone solvent, or a mixed solvent composed of a ketone solvent and a solvent selected from the group consisting of a halogenated alkane solvent and an alcohol solvent;
preferably, the solvent S1 is a binary mixed solvent composed of a ketone solvent and a solvent selected from the group consisting of a halogenated alkane solvent, water, and an alcohol solvent; the ketone solvent and the other solvent have a volume ratio of (1 to 10):1;
more preferably, the solvent S1 is a binary mixed solvent composed of acetone and a solvent selected from the group consisting of dichloromethane, ethanol, and water; the acetone and the other solvent have a volume ratio of (1 to 5):1.

6. The preparation method according to claim 5, wherein the solvent S1 is a mixed solvent composed of acetone, ethanol, and water;
preferably, the volume ratio of acetone, ethanol, and water in the solvent S1 is 1:(0.8 to 1):(0.2 to 0.7);
preferably, when the solvent S2 is a binary mixed solvent, the volume ratio of the ketone solvent to the other solvent is (1 to 10):1.

7. A pharmaceutical composition, comprising the solid dispersion as defined in any one of claims 1 to 6 and a pharmaceutically acceptable excipient.

8. The pharmaceutical composition according to claim 7, wherein the pharmaceutically acceptable excipient comprises one or more selected from the group consisting of a filler, a lubricant, and a disintegrant.

9. The pharmaceutical composition according to claim 8, wherein the filler is one selected from the group consisting of lactose, dextrin, mannitol, microcrystalline cellulose, starch, pregelatinized starch, cellulose lactose, dicalcium phosphate, and a mannitol-starch complex, or a mixture thereof obtained by mixing two or more thereof in any proportion;
preferably, the lubricant is one selected from the group consisting of talc, magnesium stearate, calcium stearate, colloidal silicon dioxide, hydrated silicon dioxide, hydrophobic silicon dioxide, sodium stearyl fumarate, polyethylene glycol, sodium stearyl fumarate, glyceryl monostearate, and hydrogenated vegetable oil, or a mixture thereof obtained by mixing two or more thereof in any proportion;
preferably, the disintegrant is one selected from the group consisting of dry starch, croscarmellose sodium, sodium carboxymethylcellulose, calcium carboxymethylcellulose, sodium carboxymethyl starch, methylcellulose, low-substituted hydroxypropyl cellulose, crospovidone, chitosan, and microcrystalline cellulose, or a mixture thereof obtained by mixing two or more thereof in any proportion;
preferably, the pharmaceutical composition comprises, by weight percentage, 30% to 50% of the solid dispersion, 20% to 50% of the filler, 1% to 15% of the disintegrant, and 1% to 15% of the lubricant;
preferably, the pharmaceutical composition comprises, by weight percentage, 35% to 45% of the solid dispersion, 35% to 45% of the filler, 1% to 10% of the disintegrant, and 1% to 10% of the lubricant;
more preferably, the pharmaceutical composition comprises, by weight percentage, 40% to 45% of the solid dispersion, 40% to 45% of the filler, 5% to 10% of the disintegrant, and 1% to 5% of the lubricant.

10. The pharmaceutical composition according to claim 9, wherein the pharmaceutical composition further comprises a disintegration promoter;
preferably, the disintegration promoter is one selected from the group consisting of sodium chloride, sodium carbonate, calcium carbonate, potassium carbonate, calcium magnesium carbonate, zinc carbonate, magnesium carbonate, ammonium carbonate, sodium glycine carbonate, sodium sesquicarbonate, sodium bicarbonate, calcium bicarbonate, potassium bicarbonate, and ammonium bicarbonate, or a mixture thereof obtained by mixing two or more thereof in any proportion;
preferably, the pharmaceutical composition comprises, by weight percentage, 30% to 50% of the solid dispersion, 20% to 50% of the filler, 1% to 15% of the disintegrant, 1% to 15% of the lubricant, and 0.1% to 10% of the disintegration promoter;
preferably, the pharmaceutical composition comprises, by weight percentage, 35% to 45% of the solid dispersion, 35% to 45% of the filler, 1% to 10% of the disintegrant, 1% to 10% of the lubricant, and 1% to 10% of the disintegration promoter;
more preferably, the pharmaceutical composition comprises, by weight percentage, 40% to 45% of the solid dispersion, 40% to 45% of the filler, 5% to 10% of the disintegrant, 1% to 5% of the lubricant, and 5% to 10% of the disintegration promoter.

11. A preparation method for the pharmaceutical composition as defined in any one of claims 7 to 9, comprising the step of mixing the solid dispersion as defined in any one of claims 7 to 9 with a pharmaceutically acceptable excipient.

12. The preparation method according to claim 11, wherein the pharmaceutical composition is prepared by a dry granulation process;
preferably, the preparation method of dry granulation comprises: weighing a formulation amount of materials, wherein the solid dispersion is not sieved, while the other excipients are sieved for later use; pre-mixing the intra-granular filler, disintegrant, glidant, and solid dispersion, followed by milling and sieving; adding the lubricant for dry granulation; then adding the extra-granular filler, disintegrant, swelling agent, and lubricant, followed by final blending and tableting.

13. Use of the solid dispersion as defined in any one of claims 1 to 4 or the pharmaceutical composition as defined in any one of claims 7 to 10 in the manufacture of a P2X4 receptor antagonist.

14. The use according to claim 13, wherein the P2X4 receptor antagonist is used to treat the following diseases: a respiratory system disease, a urinary system disease, a gynecological disease, an ophthalmic disease, a gastrointestinal disease, a proliferative disease, a cardiovascular and cerebrovascular disease, a neurodegenerative or degenerative disease, inflammation, a pain-related disease, pruritus (*e.g*., chronic pruritus), stroke, ischemic brain injury, traumatic brain injury, a musculoskeletal and connective tissue disorder, or a systemic disorder;
the respiratory system disease is, for example, a respiratory disorder, including idiopathic pulmonary fibrosis, respiratory failure, chronic obstructive pulmonary disease, asthma, bronchospasm, cough (*e.g*., acute cough, chronic cough), refractory chronic cough, or idiopathic chronic cough;
the urinary system disease is, for example, urinary incontinence, overactive bladder, dysuria, prostatic hyperplasia, cystitis (*e.g*., interstitial cystitis), bladder pain syndrome, or prostatitis;
the gynecological disease is, for example, endometriosis, primary and secondary dysmenorrhea, dyspareunia, or adenomyosis;
the ophthalmic disease is, for example, dry eye syndrome, ocular neuropathic pain, ocular trauma, or postoperative ocular pain;
the gastrointestinal disease is, for example, gastrointestinal dysfunction, irritable bowel syndrome (IBS), inflammatory bowel disease (IBD), Crohn's disease, or gastroesophageal reflux;
the proliferative disease is, for example, a tumor, such as cancer;
the cardiovascular disease is, for example, myocardial infarction, thrombosis, atherosclerosis, heart failure, or hypertension;
the neurodegenerative or degenerative disease is, for example, Parkinson's disease, Alzheimer's disease, Huntington's disease, amyotrophic lateral sclerosis, stroke, ischemic brain injury, or traumatic brain injury;
the inflammation is, for example, arthritis, rheumatoid arthritis, ankylosing spondylitis and related neuropathic pain, or gout;
the pain-related disease is, for example, hyperalgesia, allodynia, acute and chronic inflammatory and neuropathic pain, inflammatory pain, postoperative pain, visceral pain, toothache, periodontitis, premenstrual pain, endometriosis-related pain, fibrosis-related pain, central pain, burn pain, or migraine;
the pain-related disease is, for example, pain due to neuritis, pain due to poisoning, pain due to ischemic injury, pain due to interstitial cystitis, pain due to cancer, or pain due to traumatic nerve injury.
